# EUROPÄISCHE PATENTSCHRIFT

(11) **EP 3 652 746 B1**
(45) Veröffentlichungstag und Bekanntmachung des Hinweises auf die Patenterteilung: **25.10.2023**
(21) Anmeldenummer: 18739792.2
(22) Anmeldetag: 02.07.2018
(51) Int. Cl.: G16H 20/30, A61B 5/00

(54) **SYSTEM UND VERFAHREN ZUM VERBESSERN EINES KOSMETISCHEN HAARZUSTANDS**
SYSTEM AND METHOD FOR IMPROVING A COSMETIC HAIR CONDITION
SYSTÈME ET PROCÉDÉ PERMETTANT D'AMÉLIORER UN ÉTAT DES CHEVEUX D'UN POINT DE VUE COSMÉTIQUE

(30) Priorität: 10.07.2017 DE 102017211782
(43) Veröffentlichungstag der Anmeldung: 20.05.2020
(73) Patentinhaber: Henkel AG & Co. KGaA, 40589 Düsseldorf (DE)
(72) Erfinder: KUMPAN-BAHRAMI, Esther, 40547 Düsseldorf (DE)
(86) Internationale Anmeldenummer: PCT/EP2018/067838
(87) Internationale Veröffentlichungsnummer: WO 2019/011704

(56) Entgegenhaltungen:
- WO-A1-2015/190939
- WO-A1-2017/198479
- WO-A1-2018/007358
- WO-A1-2018/166749
- WO-A2-2004/073537
- WO-A2-2009/090632

## Beschreibung

Die Erfindung betrifft Haarkosmetik, insbesondere ein System zum Verbessern eines Haarzustands und ein Verfahren zum kosmetischen Behandeln von Haaren.

In vielen Bereichen des täglichen Lebens gibt es seit einiger Zeit einen Trend zu personalisierten Programmen, die auf individuelle Voraussetzungen und Bedürfnisse gezielt eingehen können, beispielsweise in einem Ernährungs- oder Gesundheitsbereich, aber auch in einem Bereich personalisierter Kosmetik. Diese kann es einem Nutzer ermöglichen, gezielt Kosmetikprodukte zu finden und/oder Pflegehinweise zu erhalten, die auf individuelle Bedürfnisse seiner Haare abgestimmt sind, und somit eine besonders hohe Wirksamkeit ermöglichen.

Für einen Nutzer kann es praktisch unmöglich sein, ein Behandlungserfolg nachzuvollziehen, weil es dem Nutzer, zum Beispiel zu Hause oder in einem Friseursalon, an Möglichkeiten fehlt, standardisiert und objektiv ein Behandlungsergebnis zu beurteilen.

Insbesondere Laien kann es an Erfahrung fehlen, wie mit geschädigtem Haar und/oder nachlassenden Haarwuchs umzugehen ist, beispielsweise welche Pflegemittel und/oder Haarwuchsmittel und/oder Behandlungsverfahren geeignet sind.

Außerdem wäre es wünschenswert, dem Nutzer, z.B. während einer sich beispielsweise über einen längeren Zeitraum erstreckenden mehrteiligen kosmetischen Haarbehandlung, eine objektive Beurteilung eines Behandlungserfolgs und/oder eines Behandlungsverlaufs zu ermöglichen. WO2015/190939 offenbart einen Haarbehandlungsapparat für das Hairstyling mit Temperatursensor umfassend eine Steuerungsvorrichtung und zusätzlicher Kühlung.

Die vorliegende Erfindung wird durch die Ansprüche 1-12 definiert.

In verschiedenen Ausführungsbeispielen wird ein System zum Verbessern eines Haarzustands bereitgestellt. Das System kann ein Kleinelektrogerät aufweisen, welches beispielsweise eine Form eines Haarreifs, Clips oder Helms aufweisen kann. Das Kleinelektrogerät kann auch ein handgehaltenes Gerät sein.

In verschiedenen Ausführungsbeispielen kann das System zum Verbessern eines Haarzustands über einen Sensor verfügen, dessen Messergebnis berücksichtigt wird bei einem Ausführen der Haarbehandlung.

In verschiedenen Ausführungsbeispielen wird ein ganzheitliches Ökosystem aus intelligenten/smarten Geräten bereitgestellt, welche von einer Problemidentifikation (Analyse) über eine Beratung (Auswertung) bis zu einer endgültigen Bereitstellung eines gewünschten Nutzens (smartes Kleinelektrogerät, gegebenenfalls in Kombination mit geeignetem gegebenenfalls individualisiertem Produkt) beispielsweise eine verbesserte Haarqualität und/oder einen verbesserten Haarwuchs bereitstellen.

In verschiedenen Ausführungsbeispielen kann ein System zum Verbessern eines Haarzustands bereitgestellt werden, beispielsweise System zum Verbessern von Haarwuchs. Das System zum Verbessern eines Haarzustands kann in verschiedenen Ausführungsbeispielen eine Sensorvorrichtung (auch als Sensoreinheit, Analysevorrichtung oder Analyseeinheit bezeichnet) und eine Behandlungsvorrichtung (auch als Kleinelektrogerät bezeichnet) aufweisen.

Die Sensorvorrichtung kann in verschiedenen Ausführungsbeispielen einen Haarstatus, auch als Haarzustand bezeichnet, ermitteln. Der Haarzustand kann einen oder mehrere Haarzustandsparameter aufweisen.

Die Sensorvorrichtung kann in verschiedenen Ausführungsbeispielen ein Smartphone, ein Haarband, einen Kamm oder Ähnliches aufweisen. Die Sensorvorrichtung kann in verschiedenen Ausführungsbeispielen mit mindestens einem Sensor ausgestattet sein. Der Sensor kann in verschiedenen Ausführungsbeispielen eine Kamera, beispielsweise in Verbindung mit einem Mikroskop, einen Nahinfrarotsensor, einen VIS-/NIR-Sensor (d.h. einen Sensor, der in einem Wellenlängenbereich empfindlich ist, der vom Bereich sichtbaren Lichts (VIS) bis zum Nahinfrarotbereich (NIR) reicht), einen Sensor zum Ermitteln von Umweltbedingungen, wie beispielsweise einen Luftfeuchtesensor, einen Temperatursensor, ein Windsensor, usw.

In verschiedenen Ausführungsbeispielen kann die Behandlungsvorrichtung eine Leuchtvorrichtung aufweisen und eingerichtet sein, mittels der Leuchtvorrichtung eine Kopfhaut des Nutzers mit Licht zu beleuchten. In verschiedenen Ausführungsbeispielen können in der Kopfhaut vorhandene Haarfollikel zu einer verstärkten Haarproduktion angeregt werden, z.B. mehr und/oder kräftigere Haare auszubilden.

In verschiedenen Ausführungsbeispielen kann eine haarwuchsfördernde Wirkung verstärkt werden mittels eines Kombinierens des Belichtens mit mindestens einer weiteren Art der Behandlung des Haars und/oder der Kopfhaut, beispielsweise mit einer Anwendung eines Haarwuchsmittels und/oder mit einer Massage der Kopfhaut und/oder mit einer Temperaturanwendung (Erwärmen und/oder Kühlen der Kopfhaut).

In verschiedenen Ausführungsbeispielen kann die Behandlungsvorrichtung eingerichtet sein, ein Haarbehandlungsmittel, z.B. ein haarkosmetisches Mittel, auf das Haar und/oder die Kopfhaut aufzubringen. Dafür kann die Behandlungsvorrichtung beispielsweise mit einer integrierten Kartusche ausgerüstet sein. In der Kartusche kann das Haarbehandlungsmittel aufgenommen sein und mittels einer Dosiervorrichtung dosiert auf das Haar aufbringbar sein, beispielsweise mittels einer Sprühvorrichtung und/oder einer Tropfvorrichtung.

Die Behandlungsvorrichtung kann in verschiedenen Ausführungsbeispielen eingerichtet sein zum Applizieren eines kosmetischen Mittels und zum Anwenden mechanischer Arbeit, zum Beispiel zum Massieren der Kopfhaut, z.B. mittels eines Luftstroms und/oder mittels Rollen, und/oder zu einem Anwenden von Temperatur, zum Beispiel als ein Eintrag von Wärme und/oder Kälte in die Kopfhaut und/oder ins Haar unter Berücksichtigung der mittels der Sensoreinheit erfassten (und ggf. ermittelten) Daten.

In verschiedenen Ausführungsbeispielen können die Analyseeinheit und das Kleinelektrogerät separat ausgeführt sein.

In verschiedenen Ausführungsbeispielen können die Analyseeinheit und das Kleinelektrogerät integriert ausgeführt sein.

In verschiedenen Ausführungsbeispielen können eine Intensität und/oder eine Dauer und/oder eine Position der Behandlung, z.B. der Beleuchtung, der mechanischen Arbeit und/oder der Temperatur, in Abhängigkeit von mittels der Analysevorrichtung übermittelten/ermittelten Daten gesteuert oder geregelt werden.

In verschiedenen Ausführungsbeispielen kann das System zum Verbessern eines Haarzustands ferner, beispielsweise als ein zusätzliches Element, eine Eingabevorrichtung und/oder eine Ausgabevorrichtung aufweisen.

Die Eingabevorrichtung kann in verschiedenen Ausführungsbeispielen eine haptische und/oder eine akustische Eingabevorrichtung sein.

Die Ausgabevorrichtung kann in verschiedenen Ausführungsbeispielen eine optische und/oder eine akustische Ausgabevorrichtung sein.

Die Eingabevorrichtung kann in verschiedenen Ausführungsbeispielen genutzt werden zum Eingeben eines Steuerbefehls (z.B. Starten, Ändern, Stoppen des Systems zum Verbessern eines Haarzustands und/oder von Teilen des Systems zum Verbessern eines Haarzustands, z.B. nur von der Behandlungsvorrichtung oder nur von der Sensorvorrichtung).

Die Ausgabevorrichtung kann in verschiedenen Ausführungsbeispielen genutzt werden für ein Ausgeben eines Analyseergebnisses und/oder ein Ausgeben einer Verfahrens- und/oder Produktempfehlung und/oder für ein Ausgeben eines Behandlungsfortschritts.

In verschiedenen Ausführungsbeispielen kann die Eingabe-und/oder Ausgabevorrichtung separat ausgeführt sein, d.h. separat von der Behandlungsvorrichtung und von der Analysevorrichtung.

In verschiedenen Ausführungsbeispielen kann die Eingabevorrichtung einen berührungsempfindlichen Bildschirm, eine Tastatur, ein Mikrofon, eine Maus oder Ähnliches aufweisen, beispielsweise als Teil eines Smartphones, Tablets, Laptops, Smart Mirrors oder Ähnliches.

In verschiedenen Ausführungsbeispielen kann die Ausgabevorrichtung einen (z.B. berührungsempfindlichen) Bildschirm, einen Lautsprecher oder Ähnliches aufweisen, beispielsweise als Teil eines Smartphones, Tablets, Laptops, Smart Mirrors oder Ähnliches.

In verschiedenen Ausführungsbeispielen kann die Eingabe- und/oder Ausgabevorrichtung einen berührungsempfindlichen Bildschirm, eine Kombination aus Bildschirm und Tastatur oder eine Kombination aus Mikrofon und/oder Lautsprecher aufweisen.

In verschiedenen Ausführungsbeispielen kann die Eingabe-und/oder Ausgabevorrichtung in die Sensorvorrichtung integriert ausgeführt sein, beispielsweise als berührungsempfindlicher Bildschirm, als eine Kombination aus Bildschirm und Tastatur, als ein Mikrofon und/oder ein Lautsprecher.

In verschiedenen Ausführungsbeispielen kann die Eingabe-und/oder Ausgabevorrichtung in die Behandlungsvorrichtung, z.B. das Kleinelektrogerät, integriert ausgeführt sein, beispielsweise als berührungsempfindlicher Bildschirm oder als eine Kombination aus Bildschirm und Tastatur, als ein Mikrofon und/oder als ein Lautsprecher.

Das Haarbehandlungsmittel, welches auf das Haar aufgebracht werden kann, kann in verschiedenen Ausführungsbeispielen ein handelsübliches Produkt sein, beispielsweise ein Haarwuchsmittel oder ein Haarpflegemittel.

In verschiedenen Ausführungsbeispielen kann das Haarbehandlungsmittel ein individualisiertes Produkt sein, beispielsweise ein individualisiertes Produkt, welches nach einer (z.B. erstmaligen) Ermittlung des mindestens einen Haarzustandsparameters (d.h. nach einer Analyse des Haars, z.B. mittels der Sensorvorrichtung) hergestellt und mitgegeben oder versendet werden kann.

In verschiedenen Ausführungsbeispielen kann das individualisierte Produkt nach der Ermittlung des Haarzustandsparameters zu Hause, am Verkaufspunkt von Haarbehandlungsmitteln oder beim Friseur hergestellt werden, beispielsweise mittels einer in das System zum Verbessern eines Haarzustands integrierten Mischvorrichtung für Haarwuchs- und/oder Pflegemittel.

In verschiedenen Ausführungsbeispielen kann die Mischvorrichtung separat bereitgestellt sein oder werden, d.h. physisch getrennt von der Behandlungsvorrichtung, von der Sensorvorrichtung und von der Eingabe- und/oder Ausgabevorrichtung ausgeführt sein.

In verschiedenen Ausführungsbeispielen kann die Mischvorrichtung in das Kleinelektrogerät integriert sein.

In verschiedenen Ausführungsbeispielen kann die Mischvorrichtung in die separate Eingabe- und/oder Ausgabevorrichtung integriert sein.

In verschiedenen Ausführungsbeispielen kann eine Verbesserung des Haarzustands durch eine additive Wirkung von Haarwuchs- und/oder Pflegemittel und Behandlungsvorrichtung (d.h. die mechanische (z.B. Massage-)Einwirkung und/oder Temperaturwirkung durch die Behandlungsvorrichtung) erzielt oder gefördert werden. Alternativ kann die Verbesserung des Haarzustands durch die synergetische Wirkung von Haarwuchs- und/oder Pflegemittel und Behandlungsvorrichtung (d.h. die mechanische Einwirkung und/oder Wärme-/Kälteeinwirkung durch die Behandlungsvorrichtung) erzielt oder gefördert werden.

Bei einer separaten Ausführung von Sensorvorrichtung, Behandlungsvorrichtung, Ein-/Ausgabevorrichtung und/oder Mischvorrichtung kann in verschiedenen Ausführungsbeispielen eine Kommunikation innerhalb des mehrteiligen Systems kabellos erfolgen.

Die kabellose Kommunikation kann in verschiedenen Ausführungsbeispielen mittels eines zentralen Kommunikationsknotenpunkts bereitgestellt werden.

In verschiedenen Ausführungsbeispielen kann die Kommunikation innerhalb einer aus den einzelnen Komponenten des Systems entsprechend einer Verfahrensabfolge gebildeten Kommunikationsreihe erfolgen.

In verschiedenen Ausführungsbeispielen kann die Kommunikation innerhalb eines aus den Komponenten des Systems gebildeten Netzwerks erfolgen.

In verschiedenen Ausführungsbeispielen wird ein ganzheitliches System bereitgestellt, welches eine standardisierte und objektive Beurteilung eines Behandlungsergebnisses ermöglicht. In verschiedenen Ausführungsbeispielen kann unter Zuhilfenahme eines breiten Daten- und Erfahrungssatzes (welcher beispielsweise mittels Zugreifens des Systems zum Verbessern eines Haarzustands auf eine Prozessor-Cloud-Architektur (kurz: Cloud) dem System zum Verbessern eines Haarzustands zugänglich gemacht sein oder werden kann) eine Optimierung eines Behandlungsergebnisses ermöglicht werden.

In verschiedenen Ausführungsbeispielen kann das System zum Verbessern eines Haarzustands als ein lernendes System ausgebildet sein. Dafür können beispielsweise Erfahrungen anderer Nutzer fortlaufend dem System zum Verbessern eines Haarzustands bereitgestellt werden, beispielsweise mittels Bereitstellens der Erfahrungen durch die anderen Nutzer an die Cloud und ggf. Weiterverarbeitens der Erfahrungen in der Cloud.

In verschiedenen Ausführungsbeispielen wird ein System bereitgestellt, welches eine Kombination aus einem haarwuchsfördernden Gerät (welches eingerichtet ist, den Haarwuchs mittels Beleuchtens der Kopfhaut mit Licht zu fördern), einer Sensorik, d.h. mindestens einen Sensor (wobei der mindestens eine Sensor in das Gerät integriert oder physisch getrennt vom Gerät, z.B. Teil einer separaten Sensorvorrichtung, sein kann), einer elektronischen Schaltkreisvorrichtung (z.B. einem Prozessor, z.B. einem Mikroprozessor) und einem Aktuator (zum Steuern oder Regeln einer Haarbehandlungsfunktionalität des Geräts, z.B. eine Beleuchtungssteuerung, eine Temperatursteuerung oder -regelung und/oder ein Steuern oder Regeln einer Massagefunktion) aufweist.

In verschiedenen Ausführungsbeispielen kann die Kombination ferner eine Ein- und/oder Ausgabevorrichtung und/oder eine Mischvorrichtung aufweisen.

Ferner wird in verschiedenen Ausführungsbeispielen ein Verfahren bereitgestellt, welches dieses System nutzt.

In verschiedenen Ausführungsbeispielen kann die elektronische Schaltkreisvorrichtung eingerichtet sein, Sensordaten vom Sensor zu empfangen, die Sensordaten auszuwerten und gegebenenfalls die ausgewerteten Sensordaten mit mindestens einer externen Datenbank (z.B. mittels einer Cloud) abzugleichen.

In verschiedenen Ausführungsbeispielen kann der Prozessor ferner eingerichtet sein, aus den ausgewerteten Sensordaten Handlungsanweisungen abzuleiten (z.B. ein Verfahrensprofil einschließlich Beleuchtungsintensität und/oder -dauer, ggf. Position für die Beleuchtung, ggf. Temperatur und/oder Art und/oder Zusammensetzung der eingesetzten Haarwuchs- und/oder Pflegemitteln).

In verschiedenen Ausführungsbeispielen kann die elektronische Schaltkreisvorrichtung ferner eingerichtet sein, gegebenenfalls eine Handlungsanweisung an den Aktuator zu übermitteln, wobei der Aktuator Teil der Behandlungsvorrichtung oder des Systems zum Verbessern eines Haarzustands sein kann.

In verschiedenen Ausführungsbeispielen kann der Aktuator die von der elektronischen Schaltkreisvorrichtung bereitgestellte Handlungsanweisung umsetzen. Eine vom Aktuator ausgeführte Handlung kann in verschiedenen Ausführungsbeispielen die Leuchtvorrichtung an-/ausschalten und/oder eine Intensität der Leuchtvorrichtung steuern oder regeln, eine mechanische Wirkung haben (z.B. als Steuern oder Regeln einer Massagefunktion, und/oder eine automatische Einstellung einer Dosiervorrichtung für ein Haarbehandlungsmittel an der Behandlungsvorrichtung), und/oder der Aktuator kann eingerichtet sein, eine automatische Temperatureinstellung zu bewirken.

In verschiedenen Ausführungsbeispielen kann der mindestens eine Sensor einen Haardichtesensor aufweisen. Der Haardichtesensor kann beispielsweise eine Kamera oder einen Kameraaufsatz aufweisen, welche/r eingerichtet sein kann, direkt an eine Haarwurzelgegend gehalten zu werden, z.B. direkt auf eine Kopfhaut aufgesetzt zu werden. Eine Haardichte kann anhand des Bildes beispielsweise anhand einer Zahl von Haaren und/oder eines Abstands zwischen den Haaren, die Haardichte zu ermitteln. Der Sensor kann das Bild der Kopfhaut als eine vergrößerte Abbildung bereitstellen, beispielsweise mit einer lupen- oder mikroskopähnlichen Vergrößerung, beispielsweise einer Vergrößerung um einen Faktor von etwa 2 bis 50, z.B. einen Faktor von etwa 10. In verschiedenen Ausführungsbeispielen kann eine Smartphonekamera mit Zoomfunktion und/oder mit einem Mikroskopaufsatz genutzt werden.

In verschiedenen Ausführungsbeispielen kann der Sensor einen Haarschädigungssensor aufweisen. Der Haarschädigungssensor kann in verschiedenen Ausführungsbeispielen eingerichtet sein, mittels einer Nahinfrarot-Spektroskopie und/oder einer Fluoreszenzspektroskopie einen Aminosäureoxidationsproduktgehalt, insbesondere einen Cysteinsäuregehalt, des Haars zu ermitteln und daraus einen Haarschädigungsgrad des Haars zu ermitteln. Der Haarschädigungssensor kann in verschiedenen Ausführungsbeispielen eingerichtet sein, mittels einer Nahinfrarot-Spektroskopie einen Haaraminosäuregehalt des Haars zu ermitteln und daraus einen Haarschädigungsgrad des Haars zu ermitteln. Der Haarschädigungssensor kann in verschiedenen Ausführungsbeispielen eingerichtet sein, während eines Kämmens des Haars erfasste akustische Emissionen aufzunehmen und anhand dessen den Haarschädigungsgrad des Haars zu ermitteln, ggf. unter Zuhilfenahme des Prozessors.

In verschiedenen Ausführungsbeispielen kann der Haarschädigungssensor einen mikroskopischen Fotosensor aufweisen. Der mikroskopische Fotosensor kann eingerichtet sein, eine Haaroberflächenrauigkeit zu ermitteln oder das Ermitteln der Haaroberflächenrauigkeit zu ermöglichen.

In verschiedenen Ausführungsbeispielen kann der Haarschädigungssensor eine Kamera aufweisen, die an ein Interferenzreflexionsmikroskop der Sensorvorrichtung gekoppelt sein kann.

In verschiedenen Ausführungsbeispielen kann der Sensor einen Haardickesensor aufweisen. Der Haardickesensor kann in verschiedenen Ausführungsbeispielen eingerichtet sein, mittels eines Lichtsensors eine Haardicke zu ermitteln. Beispielsweise kann bei dem Ermitteln der Haardicke berücksichtigt werden, dass dickeres Haar mehr Licht absorbiert. Der Haardickesensor kann dafür in verschiedenen Ausführungsbeispielen derart eingerichtet sein, dass in ein vorgegebenes Volumen eine vorbestimmte Menge an Haar, z.B. einlagig, einbringbar ist, und das Volumen von Licht mit einer vorbestimmten Intensität bestrahlt wird, wobei die Lichtmenge, die nach dem Durchstrahlen des Haars den Sensor erreicht, mittels des Sensors gemessen werden kann. Mittels des Haardickesensors kann, ggf. in Verbindung mit der elektronischen Schaltkreisvorrichtung, z.B. dem Prozessor, anhand des erfassten Lichts die Haardicke ermittelt werden.

In verschiedenen Ausführungsbeispielen kann der Haardickesensor, z.B. in einem Fall, dass der Haardickesensor eine Farbkamera aufweist, außerdem für ein Ermitteln einer Haarfarbe genutzt werden, ggf. unter Zuhilfenahme der elektronischen Schaltkreisvorrichtung.

In verschiedenen Ausführungsbeispielen kann der Haardickesensor einen Ultraschallsensor aufweisen. Der Ultraschallsensor kann eingerichtet sein, Ultraschallwellen in Richtung zu den Haaren abzustrahlen, von den Haaren reflektierte Ultraschallwellen zu erfassen und daraus, ggf. in Verbindung mit dem Prozessor, die Haardicke zu ermitteln.

In verschiedenen Ausführungsbeispielen kann der mindestens eine Sensor einen Haarlängesensor aufweisen. Der Haarlängesensor kann beispielsweise mindestens einen Lagesensor aufweisen, der eine Bestimmung einer Wegstrecke ermöglicht, welche im Haar zurückgelegt wurde. In verschiedenen Ausführungsbeispielen kann der Haarlängesensor kombiniert sein mit einem Sensor zum Berechnen einer Kämmbarkeit des Haars (siehe unten).

In verschiedenen Ausführungsbeispielen kann anstelle eines Ermittelns der Haarlänge mittels des Haarlängesensors die Haarlänge vom Nutzer bereitgestellt werden oder sein. Beispielsweise kann der Nutzer die Haarlänge selbst ausmessen und den gemessenen Haarlängewert der Behandlungsvorrichtung oder dem System zum Verbessern eines Haarzustands bereitstellen.

In verschiedenen Ausführungsbeispielen kann der Haardickesensor einen photooptischen Sensor aufweisen, bei welchem ein Bild von mindestens einem Haar aufgenommen wird.

In verschiedenen Ausführungsbeispielen kann der Haardickesensor einen thermischen Sensor aufweisen.

In verschiedenen Ausführungsbeispielen kann der Sensor einen Glätte-/Lockigkeitssensor aufweisen, der eingerichtet sein kann, eine Haarstruktur im Sinne glatten Haars bis hin zu lockigem oder krausem Haar zu ermitteln. Der Glätte-/Lockigkeitssensor kann in verschiedenen Ausführungsbeispielen eine Kamera aufweisen. Der Glätte-/Lockigkeitssensor kann in verschiedenen Ausführungsbeispielen eingerichtet sein, ggf. in Verbindung mit der elektronischen Schaltkreisvorrichtung, z.B. dem Prozessor, z.B. mittels eines Bildverarbeitungsprogramms, eine Glätte oder eine Lockigkeit des Haars zu ermitteln.

In verschiedenen Ausführungsbeispielen kann der Sensor einen Haarfeuchtigkeitssensor aufweisen. Der Haarfeuchtigkeitssensor kann in verschiedenen Ausführungsbeispielen eingerichtet sein, einen Wassergehalt des Haars zu ermitteln. Der Haarfeuchtigkeitssensor kann beispielsweise als Nahinfrarotspektroskop gestaltet sein, welches eingerichtet sein kann, Nahinfrarot-(NIR-)Absorptionsstrukturen von Wasser zu untersuchen und anhand dessen, ggf. unter Zuhilfenahme des Prozessors, die Haarfeuchtigkeit zu ermitteln.

In verschiedenen Ausführungsbeispielen kann der mindestens eine Sensor einen Kämmarbeit-Sensor aufweisen. Der Kämmarbeit-Sensor kann eingerichtet sein, einen Kraftaufwand zu erfassen (beispielsweise mittels Dehnmessstreifen), der bei einem Kämmen der Haare aufgewendet wird.

In verschiedenen Ausführungsbeispielen kann der Sensor, z.B. im Fall eines Spektrometers oder einer Kamera, eingerichtet sein, mehrere Haarzustandsparameter zu ermitteln, z.B. sowohl den Haarschädigungsgrad anhand der Cysteinsäure-Absorptionsstrukturen im NIR-Spektrum als auch die Haarfeuchtigkeit anhand der Wasser-Absorptionsstrukturen im NIR-Spektrum.

In verschiedenen Ausführungsbeispielen kann der Sensor eingerichtet sein, weitere Haarzustandsparameter zu ermitteln.

In verschiedenen Ausführungsbeispielen kann die Behandlungsvorrichtung des Systems zum Verbessern eines Haarzustands die Beleuchtungsvorrichtung aufweisen und eingerichtet sein, je nach Ergebnis der Haaranalyse (insbesondere Haardichte, aber auch Schädigung, Haardicke, Lockigkeit, Wassergehalt) gesteuert oder geregelt werden. Beispielsweise kann die Behandlungsvorrichtung oder das System zum Verbessern eines Haarzustands bei geringerer Haardichte so gesteuert oder geregelt werden, dass eine höhere haarwuchsfördernde Wirkung erzielt werden kann als bei einem Erfassen einer höheren Haardichte.

In verschiedenen Ausführungsbeispielen kann das System als eine Haarbehandlungseinheit eine Vorrichtung aufweisen, die eingerichtet ist, an der Kopfhaut mechanische Arbeit zu verrichten, beispielsweise die Kopfhaut zu massieren. Dafür kann die Vorrichtung mit Düsen zum Bereitstellen eines massierenden Luftstroms und/oder mit einem Motor, zum Beispiel zum Antreiben von massierenden Rollen, ausgerüstet sein. Mittels des (z.B. elektronisch ansteuerbaren) Motors oder den (z.B. elektronisch ansteuerbaren) Düsen kann die Massagefunktion steuer- oder regelbar sein.

In verschiedenen Ausführungsbeispielen kann die Behandlungsvorrichtung eine Aufbringvorrichtung zum Aufbringen des Haarbehandlungsmittels auf das Haar oder auf die Kopfhaut aufweisen. Die Aufbringvorrichtung kann beispielsweise einen Vorratsbehälter, auch als Tank oder Kartusche bezeichnet, und eine Dosiervorrichtung, z.B. mindestens ein (z.B. elektronisch ansteuerbares) Ventil und/oder eine (z.B. elektronisch ansteuerbare) Pumpvorrichtung aufweisen. Die Dosiervorrichtung kann als Teil des Tanks gebildet sein, und/oder als Teil eines Körpers der Behandlungsvorrichtung, auch als Vorrichtungskörper bezeichnet.

Die Kartusche kann in verschiedenen Ausführungsbeispielen wiederbefüllbar sein.

In dem Vorratsbehälter kann sich das Haar-Behandlungsmittel, z.B. ein Haarwuchsmittel und/oder ein Pflegemittel, befinden. Je nach Ergebnis der Haaranalyse (z.B. Haardichte, Schädigung, Haardicke, Lockigkeit, Wassergehalt) kann das Haarbehandlungsmittel, das z.B. eine (z.B. chemische) Zusammensetzung aufweisen kann, auf das Haar aufgebracht werden, indem die Dosiervorrichtung mittels des Aktuators betätigt wird.

In verschiedenen Ausführungsbeispielen können unterschiedliche Volumina/Mengen je nach Position (z.B. Hinterkopf, Geheimratsecken, sonstiger Kopfhautbereich oder Ansatz, Mitte, Spitzen des Haars) aufgebracht werden oder auf die Kopfhaut oder das Haar aufbringbar sein.

In verschiedenen Ausführungsbeispielen können mittels des Systems zum Verbessern eines Haarzustands zwei oder mehr Mittel in unterschiedlichen Mischungsverhältnissen je nach Position (z.B. Hinterkopf, Geheimratsecken, sonstiger Kopfhautbereich oder Ansatz, Mitte, Spitzen des Haars) oder an unterschiedlichen Positionen auf das Haar aufgebracht werden oder auf das Haar aufbringbar sein. Beispielsweise kann das Haarwuchsmittel auf die Kopfhaut aufgebracht werden (ggf. in positionsabhängiger Dosierung), und das Haarpflegemittel kann auf das Haar aufgebracht werden (ggf. in positionsabhängiger Dosierung).

In verschiedenen Ausführungsbeispielen kann ein Nutzer bereits während einer Durchführung einer Haarbehandlung Informationen über einen Verlauf der Anwendung erhalten. Ein Haarbehandlungsergebnis kann noch während der Durchführung der Haarbehandlung optimiert werden, so dass Frustrationen vermieden werden können.

In verschiedenen Ausführungsbeispielen kann ein System zum Verbessern eines Haarzustands mit einer Behandlungsvorrichtung bereit gestellt werden, bei welchem der mindestens eine Sensor Teil einer separaten kommunikationsfähigen (d.h. eine Datenaustauschvorrichtung aufweisenden) Sensorvorrichtung ist (beispielsweise ein Smartphone, das eine Kamera aufweist (ggf. mit einem Lupen- oder Mikroskopaufsatz), ein Spektrometer mit der Datenaustauschvorrichtung, ein so genannter "akustischer Kamm", welcher eingerichtet sein kann, beim Kämmen entstehende Geräusche zu erfassen und zu übermitteln, oder ähnliches).

Eine elektronische Schaltkreisvorrichtung, die einen Prozessor aufweisen kann, und ein erster Aktuator, der eine Beleuchtungsvorrichtung aufweisen kann, können in die Behandlungsvorrichtung integriert sein.

Die Beleuchtungsvorrichtung kann in verschiedenen Ausführungsbeispielen Licht mit einer haarwuchsfördernden Wellenlänge abstrahlen, z.B. in einem sichtbaren Wellenlängenbereich, z.B. zwischen etwa 400 nm und etwa 500 nm, und/oder zwischen etwa 610 nm und etwa 690 nm, und/oder in einem Nahinfrarotbereich, z.B. in einem Bereich von etwa 800 nm bis etwa 900 nm und/oder von etwa 1000 nm bis etwa 1200 nm, und/oder in jedem anderen geeigneten Wellenlängenbereich.

In verschiedenen Ausführungsbeispielen kann die Behandlungsvorrichtung als einen weiteren Aktuator eine Temperatursteuerung oder eine Temperaturregelung aufweisen, welche eine Temperatur mindestens einer beheizbaren oder kühlbaren Fläche der Behandlungsvorrichtung steuert oder regelt, mittels welcher das Haar und/oder die Kopfhaut geheizt und/oder gekühlt werden kann.

In verschiedenen Ausführungsbeispielen kann die Behandlungsvorrichtung eine Datenaustauschvorrichtung aufweisen, beispielsweise zum Empfangen von mittels des mindestens einen Sensors erfassten Messwerten und/oder zum Empfangen von Haarbehandlungsparametern, z.B. Steuerungsanweisungen.

In verschiedenen Ausführungsbeispielen kann der mindestens eine Sensor, statt Teil der separaten kommunikationsfähigen Sensorvorrichtung zu sein oder (z.B. im Fall mehrerer Sensoren) ergänzend dazu, in die Behandlungsvorrichtung integriert sein.

In verschiedenen Ausführungsbeispielen kann das System zum Verbessern eines Haarzustands zusätzlich zu der elektronischen Schaltkreisvorrichtung eine Datenverarbeitungsvorrichtung aufweisen oder eingerichtet sein, mit der Datenverarbeitungsvorrichtung Daten auszutauschen. Die Datenverarbeitungsvorrichtung kann kommunikationsfähig sein, d.h. eine Datenaustauschvorrichtung aufweisen, z.B. Teil eines Smartphones, eines Tablets, eines Laptops, eines Smart Mirrors oder Ähnliches sein, auf dem beispielsweise eine App installiert sein kann, oder beispielsweise eine Cloud oder ähnliches).

In verschiedenen Ausführungsbeispielen kann die Behandlungsvorrichtung eine Datenaustauschvorrichtung aufweisen, beispielsweise zum Empfangen von mittels der Sensorvorrichtung ermittelten Haarzustandsparameter oder von daraus ermittelten Behandlungsparametern, und/oder zum Empfangen von mittels der Datenverarbeitungsvorrichtung ermittelten Empfehlungen und/oder Steuerungsanweisungen.

Mittels eines Softwareprogramms, z.B. einer (Smartphone-)App, können gezielt Informationen, z.B. Steuerungs- oder Regelungsanweisungen, an die Behandlungsvorrichtung übertragen werden, welche eingerichtet sein kann, anhand der Steuerungs- oder Regelungsanweisungen einen Haarbehandlungsparameter zu steuern oder regeln, z.B. ein Haarbehandlungsmittel zu dosieren, eine Temperatur einzustellen, mit der die Haare behandelt werden können, usw. Ein Datenaustausch kann hierbei kabellos erfolgen, z.B. über Bluetooth, WLAN, Thread oder eine Near Field Communication Technologie (NFC-Technologie).

In einer beispielhaften Ausführungsform kann die Beleuchtungsvorrichtung mittels eines Smartphones o.ä., z.B. mittels einer App, gesteuert oder geregelt werden. Das Smartphone kann die Datenverarbeitungsvorrichtung bilden, welche Sensordaten von der Sensorvorrichtung empfängt und/oder selbst mindestens einen Sensor bereitstellt, z.B. die Smartphonekamera (ggf. im Zusammenhang mit einer geeigneten App zum Ermitteln mindestens eines Haarzustandsparameters, z.B. einer Haardichte, einer Haarfarbe o.ä.). Anhand des mindestens einen empfangenen und/oder ermittelten Haarzustandsparameters kann (z.B. mittels einer geeigneten Software, z.B. einer App) mittels des Smartphones ein Behandlungsparameter ermittelt werden. Beispielsweise kann mittels des Smartphones eine Belichtungsdauer für jede zu belichtende Stelle der Kopfhaut ermittelt, vom Smartphone an die Behandlungsvorrichtung übertragen und mittels der Behandlungsvorrichtung unter Verwendung der Leuchtvorrichtung beleuchtet werden.

In verschiedenen Ausführungsbeispielen kann ein System zum Verbessern eines Haarzustands bereitgestellt werden, welches eine Behandlungsvorrichtung aufweisen kann, und ferner eine Datenverbindung (für die Möglichkeit des Datenaustauschs ist auch der Begriff Konnektivität oder Connectivity gebräuchlich) aufweisen kann zwischen einer externen App und der Behandlungsvorrichtung. In verschiedenen Ausführungsbeispielen können Haarbehandlungsparameter bereitgestellt, z.B. vorgegeben, werden (z.B. eine Beleuchtungsdauer), wobei der bereitgestellte Parameter bezogen sein kann auf eine Haardichte, d.h. je nach Haardichte kann der Haarbehandlungsparameter einen anderen Wert aufweisen.

In verschiedenen Ausführungsbeispielen kann die Eingabevorrichtung eingerichtet sein, mindestens eine Eingabe durch den Nutzer aufzunehmen und der elektronischen Schaltkreisvorrichtung und/oder einer externen Datenverarbeitungsvorrichtung bereitzustellen.

Vom Nutzer einzugebende Parameter oder Informationen können beispielsweise Alter und/oder Geschlecht, eine Haarfarbe oder ähnliches aufweisen.

Eine oder mehrere der Informationen können in verschiedenen Ausführungsbeispielen als Teil eines Nutzerprofils abgespeichert werden, z.B. in der elektronischen Schaltkreisvorrichtung und/oder in der externen Datenverarbeitungsvorrichtung und somit bei einer späteren Verwendung wieder abrufbar sein.

Das zum Steuern oder Regeln der Haarbehandlung gesteuerte oder geregelte Element kann in verschiedenen Ausführungsbeispielen ferner eine Aufbring- oder Dosiervorrichtung für das Haarbehandlungsmittel und/oder eine Heiz- und/der Kühlvorrichtung zum Erwärmen des Haars und/oder einen Motor oder eine Düse zum Massieren der Kopfhaut aufweisen. Das gesteuerte oder geregelte Element kann in verschiedenen Ausführungsbeispielen mittels einer drahtlosen Übertragungsvorrichtung gesteuert werden, beispielsweise indem die Aufbringvorrichtung Steuerbefehle mittels der drahtlosen Übertragungsvorrichtung empfängt. Die drahtlose Übertragungsvorrichtung kann beispielsweise Teil der elektronischen Schaltkreisvorrichtung sein. Die drahtlose Übertragungsvorrichtung kann in verschiedenen Ausführungsbeispielen einen Chip oder Tag aufweisen, der die drahtlose Datenübertragung, z.B. mittels Bluetooth, WLAN, ZigBee, NFC, Wibree, Thread, WiMAX oder ähnlichem ermöglicht.

In verschiedenen Ausführungsbeispielen kann vor einem Bereitstellen einer Empfehlung an den Nutzer ein Datenabgleich vorgenommen werden zwischen der smarten Behandlungsvorrichtung und Daten, z.B. Referenzdaten, welche beispielsweise in einer Cloud hinterlegt sein können. Die Daten können in verschiedenen Ausführungsbeispielen Daten von anderen Nutzern aufweisen, welche beispielsweise denselben mindestens einen Haarzustandsparameter aufweisen, und beispielsweise entsprechende abgeleitete Empfehlungen/Maßnahmen.

In verschiedenen Ausführungsbeispielen kann die ermittelte Empfehlung für eine Herstellung eines optimalen/personalisierten Haarwuchsmittels oder sonstigen Haarpflegeprodukts und/oder für die Einleitung einer Online-Bestellung eines optimalen/personalisierten Haarwuchsmittels oder sonstigen Haarpflegeprodukts genutzt werden.

In verschiedenen Ausführungsbeispielen kann ein herkömmliches Haarbehandlungsmittel, z.B. Haarwuchsmittel verwendet werden.

Es ist vorteilhaft, dass ab einer bestimmten Füllstandsmenge eine automatische Nachbestellung des bisher eingesetzten Haarwuchsmittels oder sonstigen Haarpflegeprodukts erfolgt. Es kann bevorzugt sein, dass basierend auf einer Änderung des erfassten Haarzustandsparameters dem Nutzer ein anderes, optimaleres Haarwuchsmittel oder sonstiges Haarpflegeprodukt empfohlen wird.

In verschiedenen Ausführungsbeispielen kann eine Nutzereingabe zur optimierten Abgabe des Haarbehandlungsmittels genutzt werden. Der Nutzer kann mithilfe einer digitalen Anzeige und eines Touchscreens die gewünschte Menge an Produkt auswählen. Hierzu kann eine gespeicherte Empfehlung der Experten bereits im Gerät einprogrammiert sein, beispielsweise in einer Datenbank abgelegt (d.h. gespeichert) sein. Mittels einer vorherigen individuellen Eingabe z.B. einer Größe von zu behandelnden Flächen (z.B. nur der Hinterkopf auf einer Fläche mit einem vorgegebenen Durchmesser, Hinterkopf und Geheimratsecken, usw.) kann so eine (Mengen- und) Produktempfehlung abgegeben werden. Diese kann vom Nutzer entweder bestätigt, erweitert oder verringert werden, z.B. wenn dieser selbst schon weiß, dass er tendenziell mehr/weniger Produkt anwendet, als auf Verpackungen normalerweise angegeben.

In verschiedenen Ausführungsbeispielen wird ein Verfahren zum kosmetischen Behandeln von Haaren bereitgestellt, welches eine richtige, d.h. zu einem Bedarf an Haarwuchsförderung oder sonstiger Haarpflege passende, Behandlungs- und Produktauswahl ermöglicht.

Hierin kann Bezug genommen werden auf "die Sensoren", beispielsweise hinsichtlich einer Datenübertragung zwischen den Sensoren und einer Datenverarbeitungsvorrichtung, einer Anordnung der Sensoren, usw. Dies ist so zu verstehen, dass die Sensoren eine Gesamtheit von in der Sensorvorrichtung oder der Behandlungsvorrichtung angeordneten Sensoren aufweisen kann, z.B. eine Gesamtheit von Kamera/s, Temperatursensor/en, Mikrofon/en, usw., oder, sofern dies aus dem Kontext hervorgeht, einen Teil der genannten Sensoren.

In verschiedenen Ausführungsbeispielen kann das System zum Verbessern eines Haarzustands eine elektronische Vorrichtung aufweisen, z.B. eine mobile elektronische Vorrichtung (auch als Mobile Device bezeichnet), z.B. ein Smartphone oder ein Tablet, oder z.B. eine sonstige Datenverarbeitungsvorrichtung (z.B. einen PC). In verschiedenen Ausführungsbeispielen kann das System zum Verbessern eines Haarzustands ferner eine (ggf. weitere) externe Datenverarbeitungsvorrichtung, z.B. eine Cloud, nutzen für eine Signalauswertung, z.B. als eine Erweiterung der Signalauswertung. Dafür können in verschiedenen Ausführungsbeispielen die mittels der Sensoren erfassten Signale mit in einer Datenbank hinterlegten Signalen (auch als Vergleichssignale, Vergleichsdaten, Referenzsignale oder Referenzdaten bezeichnet) verglichen werden. In verschiedenen Ausführungsbeispielen kann anhand dessen die Haardichte, der Haarschädigungsgrad oder ein sonstiger Haarzustandsparameter eingeordnet werden, beispielsweise indem den Vergleichssignalen Haardichtewerte zugeordnet sind, und der Haardichtewert oder der sonstige Haarzustandsparameter des dem gemessenen Signal ähnlichsten Vergleichssignals den gemessenen Haaren zugeordnet wird.

Die Referenzdaten, die z.B. als eine Datenbank bereitgestellt sein können, können in verschiedenen Ausführungsbeispielen empirisch (z.B. im Labor) gewonnen sein für Haare, deren Haarzustandsparameter (z.B. Haardichte) bekannt sein kann. In verschiedenen Ausführungsbeispielen können ferner weitere Informationen über die Haare vorliegen, die als Grundlage für die Referenzdaten dienen können, z.B. Alter, Geschlecht, Haarfarbe des Nutzers, was im Zusammenhang mit einer Beurteilung, ob eine ermittelte Haardichte normal oder verbesserungsbedürftig ist, und/oder Informationen wie "viermal gebleichtes Haar - hoher Schädigungsgrad" oder "unbehandeltes Haar - keine Schädigung", im Zusammenhang mit einer Beurteilung des Haarschädigungsgrads

Ferner kann in verschiedenen Ausführungsbeispielen ein Erfassen der Referenzdaten beispielsweise mehrmals im Verlauf einer mehrteiligen Behandlung erfolgen. Wie viele Behandlungen bereits erfolgt sind kann beispielsweise als ein Behandlungsgrad parametrisiert sein.

In verschiedenen Ausführungsbeispielen kann einem Nutzer eine (z.B. der Datenbank entnommene) Zusatzinformation dahingehend bereitgestellt werden, welchem Behandlungsgrad sein Haarzustand entspricht, und/oder wie sich sein Haarzustand voraussichtlich entwickeln wird, wenn er eine bestimmte Behandlung vornimmt, z.B. eine Beleuchtungsbehandlung durchführt und/oder ein bestimmtes Mittel anwendet.

In verschiedenen Ausführungsbeispielen, z.B. bei Verwendung einer Cloud, kann die Datenbank alternativ zu einem Erzeugen im Labor mittels Nutzerdaten erzeugt werden (und beispielsweise fortlaufend ergänzt werden). In verschiedenen Ausführungsbeispielen kann die im Labor erzeugte Datenbank mittels Nutzerdaten, welche mittels der Cloud bereitgestellt werden können, ergänzt werden.

In verschiedenen Ausführungsbeispielen können die aufgezeichneten Daten, wie hierin an anderer Stelle beschrieben, mittels Softwarealgorithmen analysiert werden, um einen Haarzustandsparameter, z.B. eine Haardichte (z.B. als Zahl von Haaren pro Flächeneinheit), Haarschädigungsgrad, oder sonstigen Parameter zu ermitteln.

In verschiedenen Ausführungsbeispielen, beispielsweise wenn die Referenzdaten mittels der Cloud bereitgestellt werden, können diese einem Nutzer jederzeit zur Verfügung stehen, um als Referenzdaten für einen Vergleich genutzt zu werden.

In verschiedenen Ausführungsbeispielen können die mittels der Behandlungsvorrichtung oder mittels des Systems zum Verbessern eines Haarzustands erfassten Daten gespeichert werden, beispielsweise in einem in der Behandlungsvorrichtung integrierten Speicher und/oder in der externen Datenverarbeitungsvorrichtung, z.B. der Cloud. Die gespeicherten Daten können so abgespeichert werden, dass zumindest dem Nutzer ermöglicht ist, diese Daten als seine Daten zu erkennen. Damit kann ein Vergleichen von Haarinformationen, die beispielsweise zu verschiedenen Zeitpunkten gewonnen wurden (z.B. vor und nach einer Behandlung) miteinander ermöglicht sein.

In verschiedenen Ausführungsbeispielen kann das System zum Verbessern eines Haarzustands eine Verbindung zum Übertragen (Senden und/oder Empfangen) von Daten aufweisen, beispielsweise zwischen einem Smartphone/Tablet, welches Teil des Systems zum Verbessern eines Haarzustands sein kann, und einer Cloud, und/oder zwischen der Behandlungsvorrichtung und dem Smartphone/Tablet, und/oder zwischen der Behandlungsvorrichtung und der Cloud, und/oder zwischen der Sensorvorrichtung und der Behandlungsvorrichtung und/oder zwischen der Sensorvorrichtung und dem Smartphone/Tablet, und/oder zwischen der Sensorvorrichtung und der Cloud.

In verschiedenen Ausführungsbeispielen kann die Analyse durch die Behandlungsvorrichtung, beispielsweise mittels der Schaltkreisvorrichtung, erfolgen, und ein Analyseergebnis kann zum Bereitstellen des Analyseergebnisses an eine Anzeigevorrichtung übertragen werden, beispielsweise an ein Display, einen Lautsprecher, ein Smartphone oder ähnliches.

In verschiedenen Ausführungsbeispielen können die Daten übertragen werden auf/an eine Datenverarbeitungsvorrichtung, z.B. auf ein Smartphone mit App, an eine Cloud, usw. Nach der Datenübertragung auf die Datenverarbeitungsvorrichtung kann mittels dieser die Untersuchung der Daten ausgeführt werden, beispielsweise zum Ermitteln eines Steuerungs- oder Regelungsparameters. Die Datenverarbeitungsvorrichtung kann in verschiedenen Ausführungsbeispielen Teil des Systems zum Verbessern eines Haarzustands sein, z.B. in einem Fall, dass die Datenverarbeitungsvorrichtung, z.B. das Smartphone o.ä., Teil der Sensorvorrichtung und/oder der Ein- und/oder Ausgabevorrichtung oder ein eigenständiger Teil des Systems zum Verbessern eines Haarzustands ist (auch als integrierte Datenverarbeitungsvorrichtung bezeichnet).

In verschiedenen Ausführungsbeispielen kann die Datenverarbeitungsvorrichtung eine externe Datenverarbeitungsvorrichtung sein, beispielsweise die Cloud.

In verschiedenen Ausführungsbeispielen kann der Steuerungs- oder Regelungsparameter oder die Empfehlung direkt mittels des Systems zum Verbessern eines Haarzustands, z.B. der Behandlungsvorrichtung und/oder der Sensorvorrichtung und/oder der Eingabe- oder Ausgabevorrichtung und/oder der sonstigen integrierten Datenverarbeitungsvorrichtung, ermittelt sein oder werden, d.h. die elektronische Schaltkreisvorrichtung und/oder die integrierte Datenverarbeitungsvorrichtung kann eingerichtet sein, den Steuerungs-/Regelungsparameter und ggf. weitere Informationen selbst (auch als direkt bezeichnet) zu ermitteln. Beispielsweise kann die elektronische Schaltkreisvorrichtung eine Datenverarbeitungsvorrichtung sein oder aufweisen, z.B. kann sie mit einem Speicher und einem Prozessor, z.B. einem Mikroprozessor, ausgerüstet sein, welche eingerichtet sein können, z.B. mittels einer Programmierung, die Sensordaten zu empfangen und die Behandlungsvorrichtung zu steuern oder zu regeln, und ggf. um dem Nutzer Informationen bereitzustellen. Beispielsweise können die Sensordaten wie oben beschrieben mit einer Datenbank verglichen werden.

In verschiedenen Ausführungsbeispielen kann die elektronische Schaltkreisvorrichtung eingerichtet sein, den Steuerungs- oder Regelungsparameter oder die Empfehlung, z.B. Produkt- oder Behandlungsempfehlung, indirekt zu ermitteln. Beispielsweise kann die elektronische Schaltkreisvorrichtung (z.B. zusätzlich zu einem Speicher und einem Prozessor, z.B. einem Mikroprozessor) mit einer Datenübertragungsvorrichtung ausgerüstet sein, welche eingerichtet sein kann, die von der elektronische Schaltkreisvorrichtung empfangenen Sensordaten an eine externe Datenverarbeitungsvorrichtung, z.B. an einen Computer, z.B. eine Cloud, zu übermitteln, mittels derer, beispielsweise wie oben für das Ermitteln der Steuerungs- oder Regelungsparameter oder der Empfehlung mittels der elektronischen Schaltkreisvorrichtung beschrieben, der Steuerungs- oder Regelungsparameter oder die Empfehlung ermittelt werden kann, um den Steuerungs- oder Regelungsparameter oder die Empfehlung bereitzustellen, beispielsweise mittels Übertragens an die Behandlungsvorrichtung und/oder an die Anzeigevorrichtung und/oder mittels Übertragens der Empfehlung zurück an die elektronische Schaltkreisvorrichtung (z.B. mittels der Datenübertragungsvorrichtung). In verschiedenen Ausführungsbeispielen kann die Datenübertragung mehrstufig erfolgen, beispielsweise indem die Sensordaten von der Schaltkreisvorrichtung zunächst an die Anzeigevorrichtung (z.B. ein Smartphone, ein Tablet o.ä.) übermittelt werden, und die Anzeigevorrichtung die Sensordaten an die externe Datenverarbeitungsvorrichtung (z.B. die Cloud) überträgt.

In verschiedenen Ausführungsbeispielen wird ein System zum Verbessern eines Haarzustands bereitgestellt. Das System kann eine Sensorvorrichtung mit mindestens einem Sensor zum Erfassen eines Haarzustandsparameters und
eine Behandlungsvorrichtung aufweisen. Die Behandlungsvorrichtung kann eine Leuchtvorrichtung zum Beleuchten einer Kopfhaut eines Nutzers mit Licht und
eine elektronische Schaltkreisvorrichtung, welche mit der Sensorvorrichtung gekoppelt ist zum Empfangen des erfassten Haarzustandsparameters und/oder eines mittels der Sensorvorrichtung aus dem Haarzustandsparameter ermittelten Behandlungsparameters aufweisen, wobei die elektronische Schaltkreisvorrichtung eingerichtet sein kann, basierend auf dem empfangenen erfassten Haarzustandsparameter und/oder dem Behandlungsparameter ein Beleuchten, mittels der Beleuchtungsvorrichtung, der Kopfhaut des Nutzers mit Licht zu steuern.

In verschiedenen Ausführungsbeispielen kann die Behandlungsvorrichtung einen Haarreif, einen Haarclip oder einen Helm aufweisen.

In verschiedenen Ausführungsbeispielen kann der mindestens eine Sensor mindestens einen aufweisen aus einer Gruppe von Sensoren, die Gruppe aufweisend: eine Kamera zum Aufnehmen eines Bildes mittels sichtbaren Lichts, UV-Lichts und/oder Nahinfrarotlichts zum Ermitteln einer Haardichte, eines Haarschädigungsgrads, einer Lockigkeit, einer Haarfarbe und/oder einer Haardicke, ein Mikroskop zum Ermitteln eines Haarschädigungsgrads und/oder einer Haardicke, ein Spektrometer zum Aufnehmen eines Spektrums von sichtbarem Licht, UV-Licht und/oder Nahinfrarotlicht zum Ermitteln eines Haarschädigungsgrads und/oder eines Haarfeuchtigkeitsgehalts, und einen akustischen Sensor zum Ermitteln eines Haarschädigungsgrads.

In verschiedenen Ausführungsbeispielen können die Sensorvorrichtung und die Behandlungsvorrichtung separate Vorrichtungen sind.

In verschiedenen Ausführungsbeispielen können zumindest ein Teil der Sensorvorrichtung und die Behandlungsvorrichtung eine integrierte Einheit bilden.

In verschiedenen Ausführungsbeispielen kann die elektronische Schaltkreisvorrichtung eine kabellose Datenaustauschvorrichtung aufweisen.

In verschiedenen Ausführungsbeispielen kann die Behandlungsvorrichtung mindestens ein weiteres Behandlungselement aufweisen, wobei das Behandlungselement eine Aufbringvorrichtung für ein kosmetisches Behandlungsmittel, eine Massagevorrichtung, eine Heizvorrichtung und/oder eine Kühlvorrichtung aufweisen kann, und wobei die elektronische Schaltkreisvorrichtung ferner eingerichtet sein kann, basierend auf dem empfangenen erfassten Haarzustandsparameter und/oder dem Behandlungsparameter das kosmetische Behandlungsmittel aufzubringen, eine Massage vorzunehmen, die Kopfhaut und/oder das Haar zu erwärmen und/oder die Kopfhaut und/oder das Haar zu kühlen.

In verschiedenen Ausführungsbeispielen kann das System ferner eine Eingabevorrichtung und/oder eine Ausgabevorrichtung aufweisen.

In verschiedenen Ausführungsbeispielen kann die Eingabevorrichtung einen berührungsempfindlichen Bildschirm und/oder eine Tastatur und/oder eine Maus und/oder ein Mikrofon aufweisen, und/oder die Ausgabevorrichtung kann einen Bildschirm und/oder einen Lautsprecher aufweisen.

In verschiedenen Ausführungsbeispielen wird ein Verfahren zum Verbessern eines kosmetischen Haarzustands eines Nutzers bereitgestellt. Das Verfahren kann vor einem Behandeln oder während des Behandelns mittels eines Systems gemäß verschiedenen Ausführungsbeispielen ein Erfassen mindestens eines Haarzustandsparameters mittels des mindestens einen Sensors aufweisen, und ein Beleuchten einer Kopfhaut des Nutzers mittels der Leuchtvorrichtung basierend auf dem empfangenen erfassten Haarzustandsparameter und/oder dem daraus ermittelten Behandlungsparameter.

In verschiedenen Ausführungsbeispielen kann das Verfahren ferner ein Behandeln des Haars mittels des mindestens einen weiteren Behandlungselements basierend auf dem empfangenen erfassten Haarzustandsparameter und/oder dem daraus ermittelten Behandlungsparameter aufweisen.

In verschiedenen Ausführungsbeispielen kann das Behandeln mittels des mindestens einen weiteren Behandlungselements ein Aufbringen eines kosmetischen Behandlungsmittels auf das Haar oder die Kopfhaut, ein Massieren der Kopfhaut, ein Erwärmen der Kopfhaut und/oder des Haars und/oder ein Kühlen der Kopfhaut und/oder des Haars aufweisen.

In verschiedenen Ausführungsbeispielen kann das Ermitteln des mindestens einen Behandlungsparameters aus dem mindestens einen Haarzustandsparameter in der elektronischen Schaltkreisvorrichtung erfolgen.

In verschiedenen Ausführungsbeispielen kann das Ermitteln des mindestens einen Behandlungsparameters aus dem mindestens einen Haarzustandsparameter in der elektronischen Schaltkreisvorrichtung erfolgen.

In verschiedenen Ausführungsbeispielen können das Erfassen des Haarzustands und das Beleuchten der Kopfhaut wiederholt ausgeführt werden, wobei das Erfassen des Haarzustands bei jeder wiederholten Ausführung an einer im Wesentlichen gleichen Stelle des Haars und/oder der Kopfhaut ausgeführt werden kann.

Ausführungsbeispiele der Erfindung sind in den Figuren dargestellt und werden im Folgenden näher erläutert.

Es zeigen
Figur 1A bis 1J jeweils eine schematische Darstellung eines Systems zum Verbessern eines Haarzustands gemäß verschiedenen Ausführungsbeispielen;
Figur 2A und 2B jeweils eine schematische Darstellung einer Anwendung eines Systems zum Verbessern eines Haarzustands gemäß verschiedenen Ausführungsbeispielen; und
Figur 3 ein Flussdiagramm eines Verfahrens zum Verbessern eines kosmetischen Haarzustands gemäß verschiedenen Ausführungsbeispielen.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

Sofern nicht ohnehin "Steuern oder Regeln" genannt ist, sind die Begriffe "Steuern", "gesteuertes Element" usw. hierin, sofern es nicht anders beschrieben oder aus dem Kontext anders zu verstehen ist, als "Steuern oder Regeln", "gesteuertes oder geregeltes Element" usw. zu verstehen.

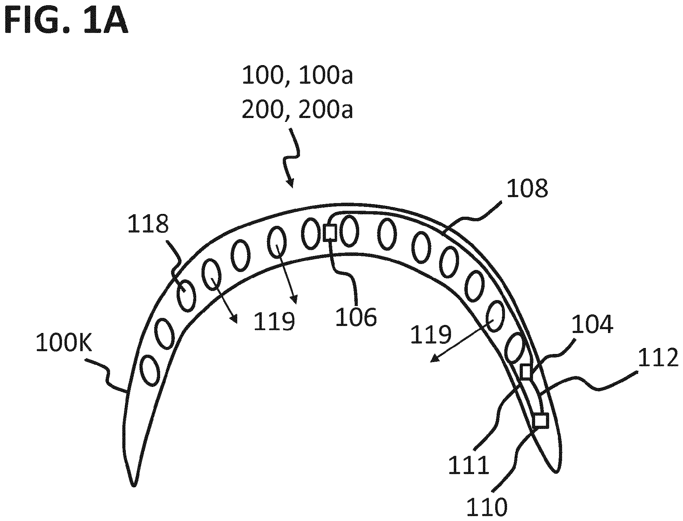

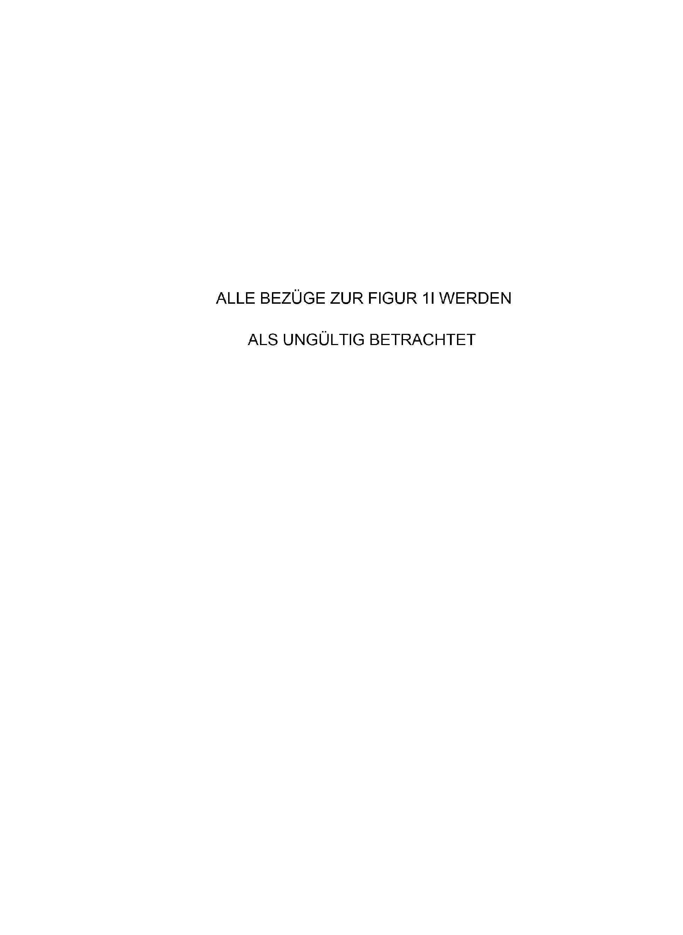

FIG. 1A bis 1J zeigen jeweils eine schematische Darstellung eines Systems 200 zum Verbessern eines Haarzustands gemäß verschiedenen Ausführungsbeispielen, wobei unterschiedliche Ausführungsbeispiele mit nachgestellten Kleinbuchstaben gekennzeichnet sind.

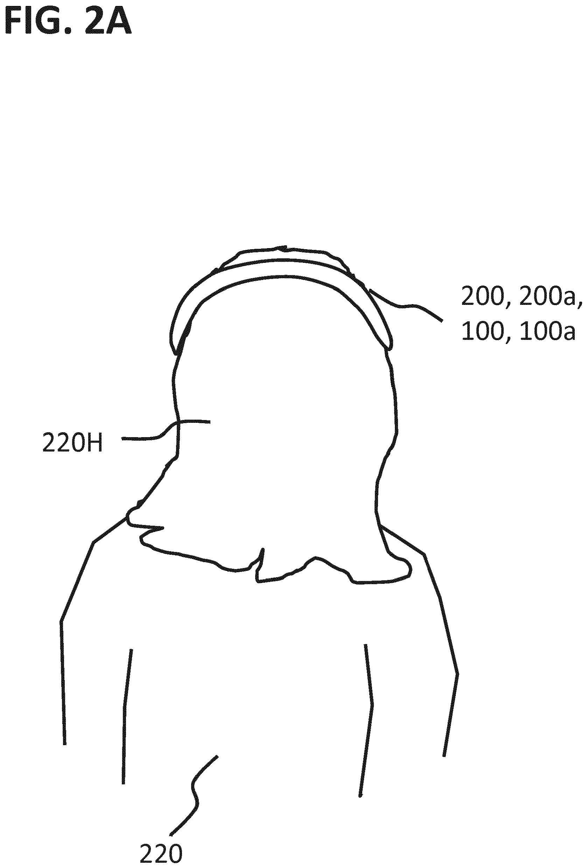

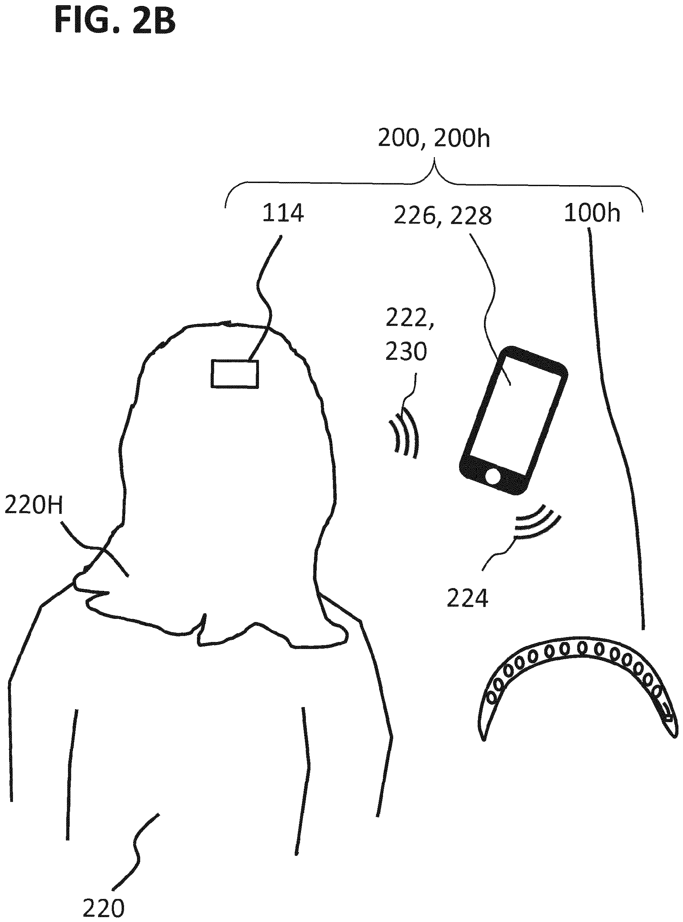

Figur 2A und 2B zeigen jeweils eine schematische Darstellung einer Anwendung eines Systems 200 zum Verbessern eines Haarzustands gemäß verschiedenen Ausführungsbeispielen.

In verschiedenen Ausführungsbeispielen weist das System 200 zum Verbessern eines Haarzustands eine Behandlungsvorrichtung 100 (verschiedene Ausführungsbeispiele sind als 100a, 100b usw. gekennzeichnet) auf.

In verschiedenen Ausführungsbeispielen kann die Behandlungsvorrichtung 100 eine im oder am Vorrichtungskörper 100K angeordnete elektronische Schaltkreisvorrichtung 104 aufweisen.

Die Behandlungsvorrichtung 100 kann einen Vorrichtungskörper 100K aufweisen. Der Vorrichtungskörper 100K kann aus einem festen Material, z.B. Kunststoff oder Metall, gebildet sein oder ein solches Material aufweisen.

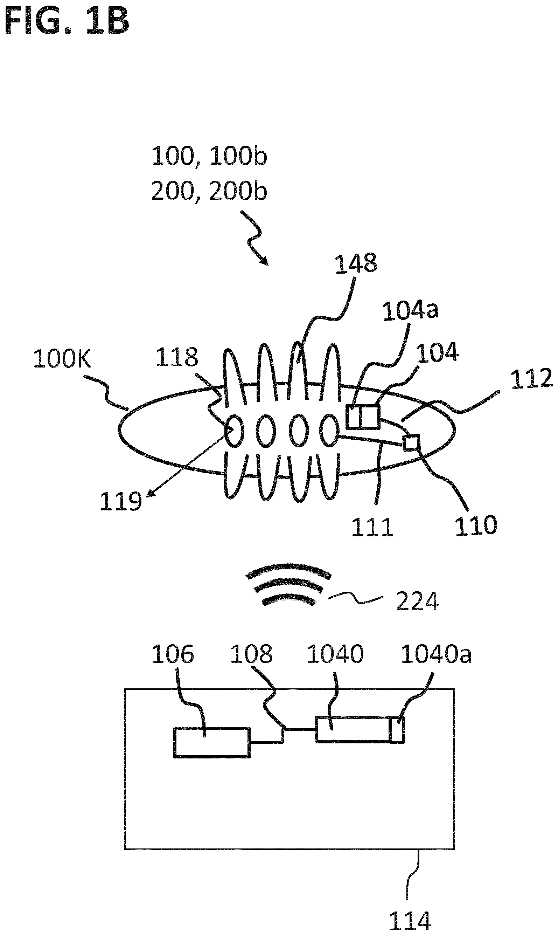

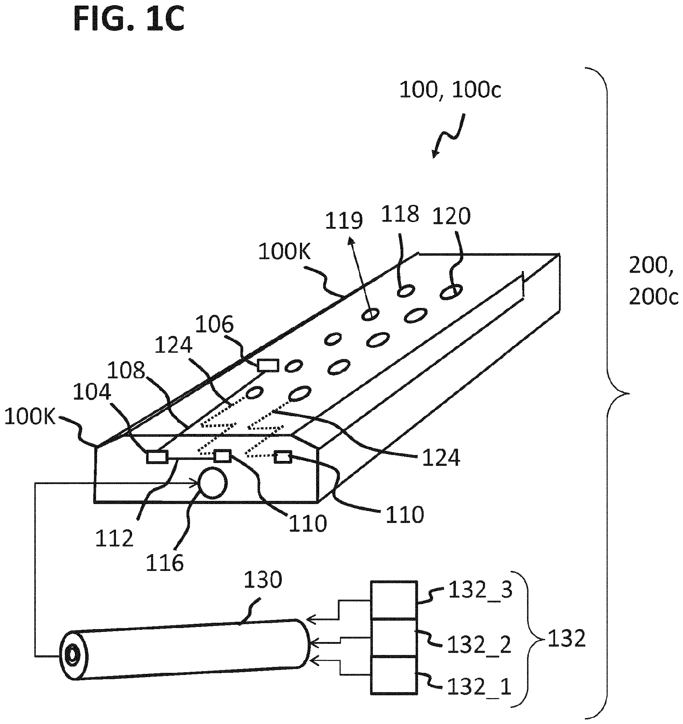

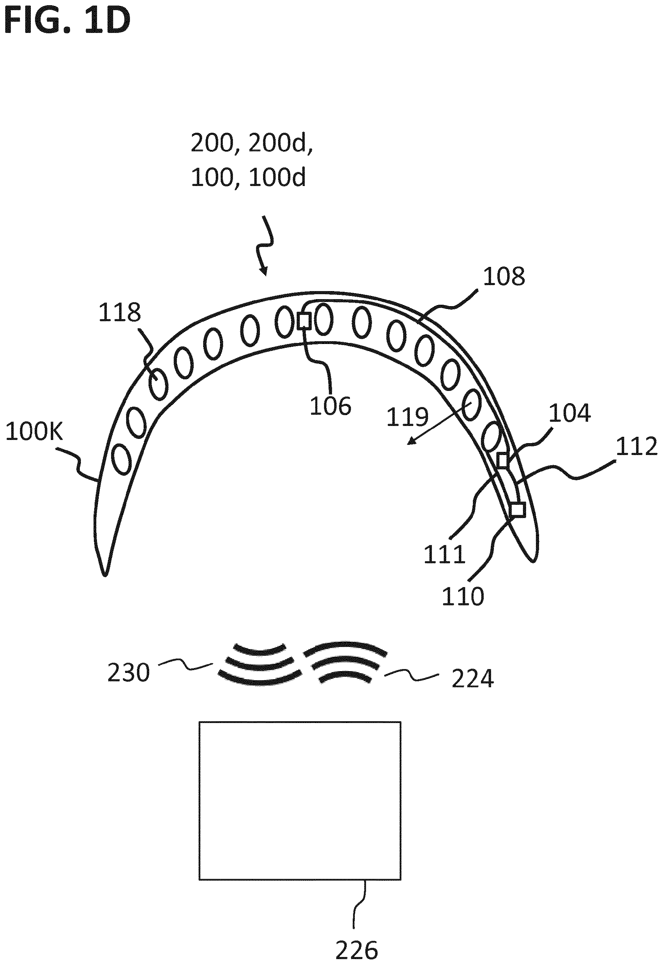

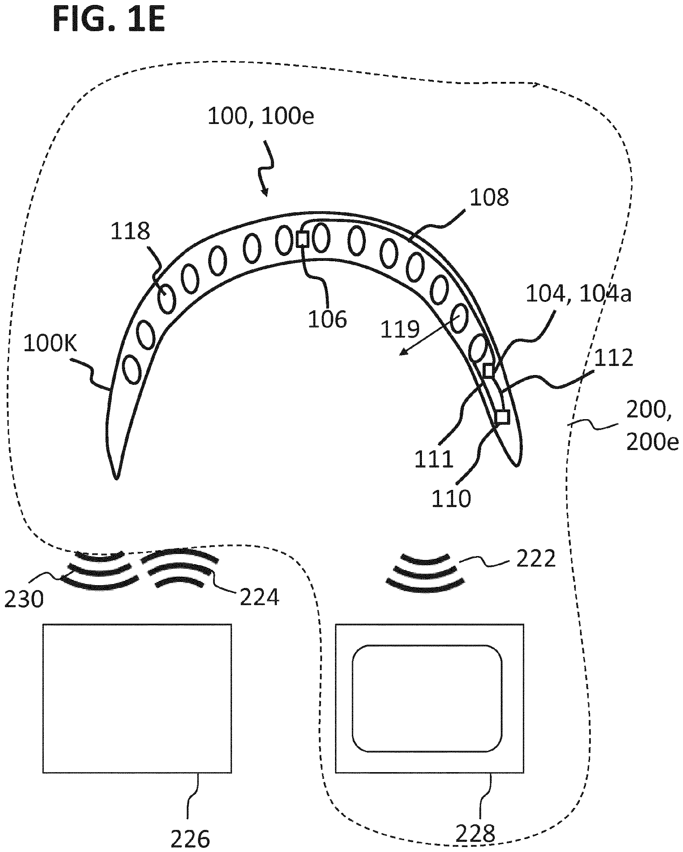

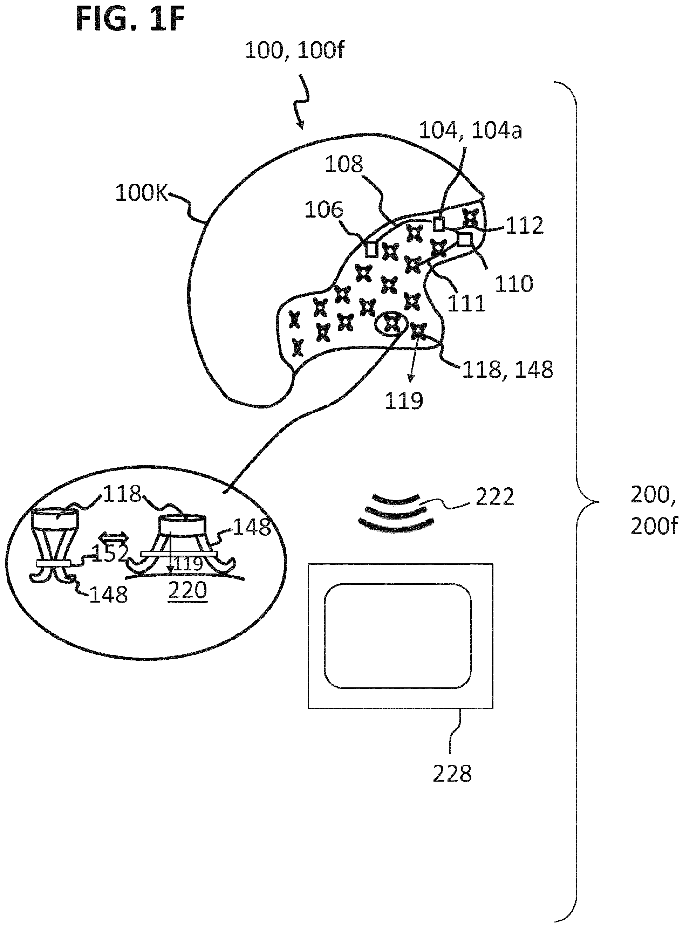

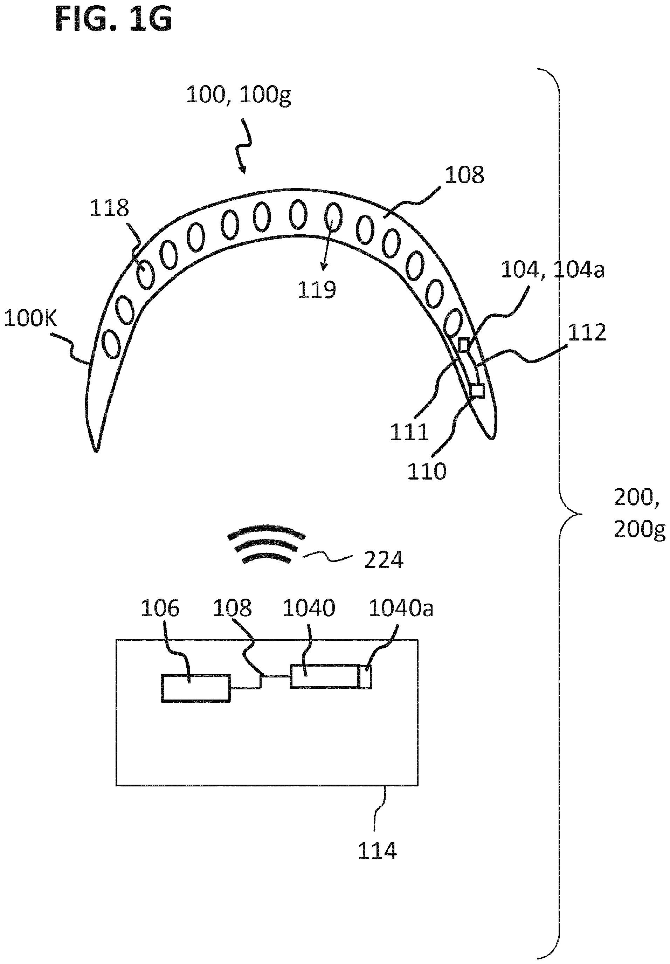

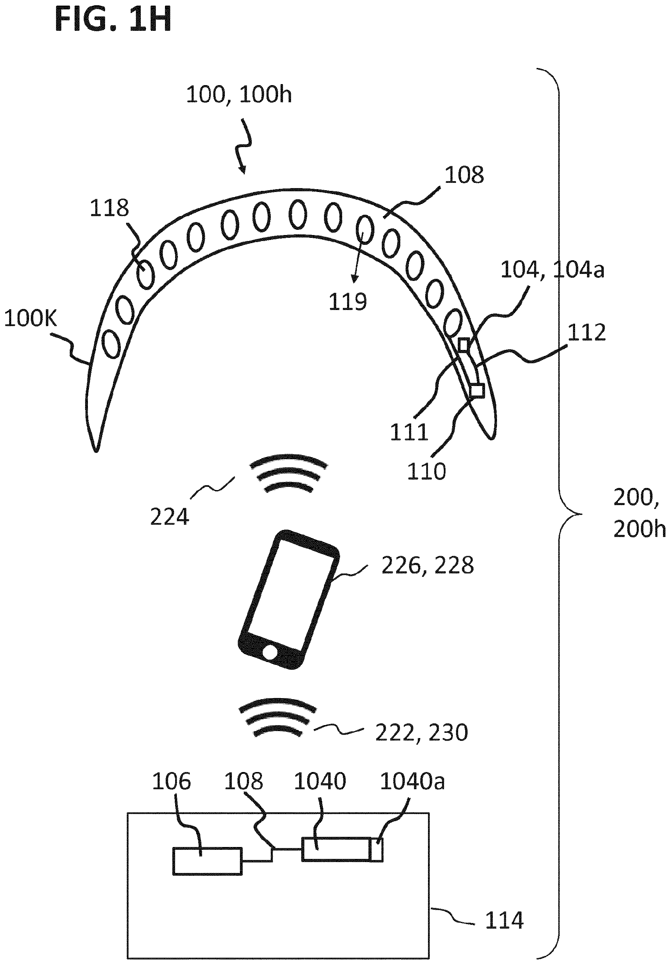

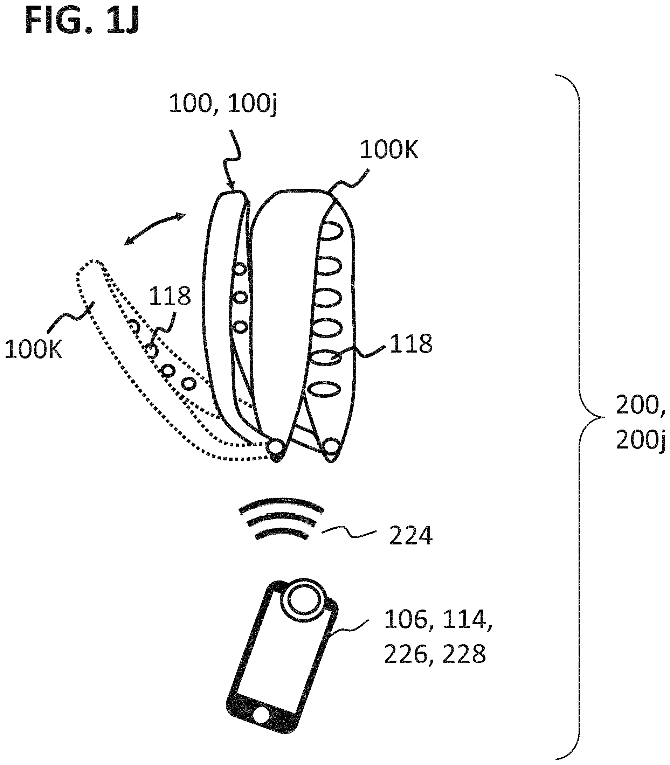

Der Vorrichtungskörper 100K kann so gestaltet sein, dass er an einem Kopf eines Nutzers 220 anbringbar ist und/oder entlang des Kopfes bewegbar ist. Beispielhafte Gestaltungen können haarreifartig sein wie z.B. in FIG. 1A, 1D, 1E, 1G, 1H, 2A, 2B oder mehrteilig haarreifartig sein wie in FIG. 1J, kammartig wie in FIG. 1B, blockartig wie in FIG. 1C oder helmartig wie in FIG. 1F, oder mit jeder beliebigen anderen geeigneten Form, die es ermöglicht, die Behandlung mittels der Vorrichtung an Haar und/oder Kopfhaut des Nutzers 220 vorzunehmen.

In verschiedenen Ausführungsbeispielen kann die Behandlungsvorrichtung 100 ferner eine steuer- oder regelbare Leuchtvorrichtung 118 aufweisen, welche in den Vorrichtungskörper 100K integriert sein kann. Die Leuchtvorrichtung 118 kann beispielsweise eine Mehrzahl von Leuchten, z.B. LED-Leuchten aufweisen. Die Leuchtvorrichtung 118 kann so eingerichtet sein, dass bei einer Verwendung der Behandlungsvorrichtung Licht 119 auf die Kopfhaut des Nutzers 220 gestrahlt werden kann.

Das Licht 119 kann, wie oben beschrieben, eine Wellenlänge oder einen Wellenlängenbereich aufweisen, der für ein Stimulieren von Haarwuchs geeignet ist. In verschiedenen Ausführungsbeispielen kann Licht 119 mit verschiedenen dieser Wellenlängen oder -bereiche in der Behandlungsvorrichtung 100 in Kombination verwendet werden. Vorzugsweise weist das Licht 119 eine Wellenlänge im Bereich von 640 bis 780 nm auf. Ganz besonders bevorzugt weist das Licht 119 eine konstante Wellenlänge von 655 nm (+/- 5) auf.

In verschiedenen Ausführungsbeispielen kann an der Haarbehandlungsvorrichtung 100 eine Mehrzahl von Haarteilern 148 angeordnet sein, welche dafür eingerichtet sein können, relativ dichtes Haar so zu teilen, dass das Licht 119 die Kopfhaut relativ ungehindert erreichen kann. Die Haarteiler 148 können, wie in FIG. 1B dargestellt, kammartig oder bürstenartig gebildet sein, so dass bei einem Kämmen/Bürsten (ggf. in beide Richtungen) die Haarteiler 148 das Haar 220 jeweils in Zwischenräumen zwischen den in einer Längsrichtung der Bürste benachbarten Haarteilern 148 anordnen, so dass jeweils in Querrichtung dem Haarteiler 148 benachbarte Leuchtelemente (z.B. LEDs) freiliegen. Die Haarteiler 148 sind der Übersichtlichkeit halber in manchen der Figuren nicht dargestellt, können aber im Prinzip bei jedem der Ausführungsbeispiele genutzt werden.

Die Haarteiler 148 in FIG. 1F können in verschiedenen Ausführungsbeispielen so gestaltet sein, dass auch bei einer stationären Anordnung der Behandlungsvorrichtung 100f, wie sie bei einem Helm erzielbar ist, ein Bereich zwischen der Leuchtvorrichtung 118 (z.B. LED-Elementen) und der Kopfhaut des Nutzers 220 von Haaren 220H freigelegt wird, damit das Licht 119 die Kopfhaut ungehindert erreichen kann.

In FIG. 1F sind Leuchtvorrichtung 118 und Haarteiler 148 vergrößert in zwei Konfigurationen dargestellt, links in einer Ruhekonfiguration und rechts während des Beleuchtens der Kopfhaut des Nutzers 220 mit dem Licht 119, auch als Anwendungskonfiguration bezeichnet. Eine Rückstellvorrichtung 152 kann eingerichtet sein, die Haarteiler 148 in die Ruheposition (zurück) zu führen, wenn die Behandlungsvorrichtung 200f nicht mit der Kopfhaut des Nutzers 220 in Kontakt ist, und von der Ruhekonfiguration in die Anwendungskonfiguration bringbar zu sein, wenn die Behandlungsvorrichtung 200f zum Behandeln der Kopfhaut (und ggf. des Haars 220H) am Kopf des Nutzers 220 angeordnet wird. Beispielsweise können sich die Haarteiler 148 beim Bringen in die Anwendungskonfiguration radial von der Leuchtvorrichtung 118 weg bewegen und dabei Haare 220H von der Leuchtvorrichtung 118 weg bewegen.

In verschiedenen Ausführungsbeispielen kann die Behandlungsvorrichtung mindestens ein weiteres Behandlungselement aufweisen, wobei das Behandlungselement eine Aufbringvorrichtung 120 (siehe FIG. 1C) für ein kosmetisches Behandlungsmittel, eine Massagevorrichtung, eine Heizvorrichtung und/oder eine Kühlvorrichtung aufweisen kann, beispielsweise wie oben beschrieben. Die elektronische Schaltkreisvorrichtung 104 kann ferner eingerichtet sein, basierend auf dem empfangenen erfassten Haarzustandsparameter und/oder dem Behandlungsparameter das kosmetische Behandlungsmittel aufzubringen, eine Massage vorzunehmen, die Kopfhaut und/oder das Haar zu erwärmen und/oder die Kopfhaut und/oder das Haar zu kühlen.

In verschiedenen Ausführungsbeispielen kann das System 200 zum Verbessern eines Haarzustands eine Sensorvorrichtung 114 mit mindestens einem Sensor 106 aufweisen zum Erfassen mindestens eines Haarzustandsparameters. Der mindestens eine Sensor 106 kann einen oder mehrere der oben beschriebenen Sensoren 106 und/oder andere/weitere Sensoren 106 aufweisen.

Der mindestens eine Haarzustandsparameter kann, wie oben ausgeführt, z.B. eine Haardichte, eine Haarfarbe, eine Haarfeuchtigkeit, einen Haarschädigungsgrad, eine Haardicke, eine Lockigkeit oder ähnliches aufweisen.

In verschiedenen Ausführungsbeispielen kann der mindestens eine Sensor 106 eingerichtet sein, mehr als einen Parameter zu erfassen, beispielsweise kann der Sensor 106 ein NIR-Spektrometer aufweisen, welches eingerichtet sein kann, sowohl Parameter zum Ermitteln der Haarfeuchtigkeit als auch Parameter zum Ermitteln des Haarschädigungsgrads zu erfassen, und/oder der Sensor 106 kann eine Kamera aufweisen, welche (z.B. mittels einer Software) eingerichtet sein kann, Parameter zum Ermitteln der Lockigkeit zu ermitteln.

In verschiedenen Ausführungsbeispielen, wie in FIG. 1A, 1C, 1D, 1E, 1F und 2A dargestellt ist, kann der Sensor 106 im Vorrichtungskörper 100K angeordnet sein, beispielsweise versiegelt eingebaut sein. Damit kann ermöglicht sein, dass die Behandlungsvorrichtung 100 unempfindlich ist gegen Feuchtigkeit (insbesondere auch gegen eine Feuchtigkeit eines ggf. aufzubringenden Haarbehandlungsmittels) und Schmutz.

Ferner kann mittels des Versiegelns erreicht werden, dass die Behandlungsvorrichtung gereinigt werden kann, ohne den Sensor 106 oder eine andere Vorrichtung zu beschädigen. Der Sensor 106 kann beispielsweise bei einem Spritzgießen des Vorrichtungskörpers 100K mit eingegossen werden.

In verschiedenen Ausführungsbeispielen, z.B. wenn der mindestens eine Sensor 106 einen optischen Sensor aufweist oder daraus besteht, kann der Vorrichtungskörper 100K zwischen dem Sensor 106 und einer Oberfläche des Vorrichtungskörpers 100K transparent sein. In verschiedenen Ausführungsbeispielen kann, sofern das zweckdienlich ist, der Sensor 106 derart im Vorrichtungskörper 100 angeordnet sein, dass er dem Haar 220H des Nutzers 220 bei einer üblichen Anordnung der Haare an oder in der Behandlungsvorrichtung 100 zugewandt ist und/oder mit dem Haar 220H in Kontakt ist.

In verschiedenen Ausführungsbeispielen, in welchen der Sensor 106 im Vorrichtungskörper 100K angeordnet ist und der Vorrichtungskörper 100K in einer im Wesentlichen immer gleichen Position am Kopf des Nutzers 220 anordenbar ist, wie beispielsweise der Helm aus FIG. 1F oder der Haarreif, dann das Erfassen der Sensordaten mittels des integrierten Sensors 106 an einer im Wesentlichen immer gleichen Stelle des Kopfes vorgenommen werden, so dass eine zuverlässige Behandlungsverlaufskontrolle ermöglicht wird.

In verschiedenen Ausführungsbeispielen, wie in FIG. 1B, 1G, 1H, 1J und 2B dargestellt, kann der Sensor 106 nicht in der Behandlungsvorrichtung 100 integriert sein, sondern Teil einer separaten Sensorvorrichtung 114 sein. Die separate Sensorvorrichtung 114 kann, wie in FIG. 1B, 1G, 1H und 2B dargestellt, in verschiedenen Ausführungsbeispielen nur dem Erfassen, ggf. Bearbeiten und Übermitteln der erfassten und/oder bearbeiteten Daten dienen.

In verschiedenen Ausführungsbeispielen kann die Sensorvorrichtung 114 außerdem mindestens eine weitere Funktion erfüllen, d.h. die Sensorvorrichtung 114 kann, wie in FIG. 1J beispielhaft dargestellt, mit einer weiteren Vorrichtung des Systems integriert sein, beispielsweise mit einer Ein- und/oder Ausgabevorrichtung. In FIG. 1J kann ein Smartphone beispielsweise als Sensor genutzt werden, z.B. mittels seiner Kamera, und sein berührungsfähiger Bildschirm kann als Ein- und Ausgabevorrichtung genutzt werden. Außerdem kann die elektronische Schaltkreisvorrichtung des Smartphones, die eine Prozessor und einen Speicher aufweisen kann, eingerichtet sein, eine Auswertung der aufgenommenen Daten (z.B. Bilder) vorzunehmen, und z.B. aus einem Bild, welches Haar 220H des Nutzers 220 zeigt, eine Haarfarbe o.ä. ermitteln. Der ermittelte Haarzustandsparameter kann der Behandlungsvorrichtung übermittelt werden zum Steuern oder Regeln der Behandlungsvorrichtung.

Die externe Sensorvorrichtung 114 kann eine elektronische Schaltkreisvorrichtung 1040 und eine kabellose Datenaustauschvorrichtung 1040a aufweisen, welche ähnlich oder gleich der elektronischen Schaltkreisvorrichtung 104 und der kabellose Datenaustauschvorrichtung 104a aus den vorherigen Ausführungsbeispielen sein können.

Die elektronische Schaltkreisvorrichtung 104 kann in verschiedenen Ausführungsbeispielen mit dem mindestens einen Sensor 106 gekoppelt sein, beispielsweise mittels einer Verbindung 108, zum Empfangen des erfassten Sensorwerts. Beim Vorhandensein einer Mehrzahl von Sensoren 106 kann die elektronische Schaltkreisvorrichtung 104 eine eigene Kopplung zu jedem der Sensoren 106 aufweisen. Die Kopplung kann in verschiedenen Ausführungsbeispielen eine elektrisch leitende Verbindung, eine (Glas-)faserverbindung und/oder eine kabellose Verbindung aufweisen oder sein. Die elektronische Schaltkreisvorrichtung 104 kann zum Empfangen des mindestens einen Sensorwerts von dem mindestens einen Sensor 106 eingerichtet sein.

In verschiedenen Ausführungsbeispielen kann die elektronische Schaltkreisvorrichtung 104 eine Datenverarbeitungsvorrichtung sein oder aufweisen, z.B. kann sie mit einem Speicher und einem Prozessor, z.B. einem Mikroprozessor, ausgerüstet sein, welche eingerichtet sein können, z.B. mittels einer Programmierung, die Daten vom Sensor 106 zu empfangen und daraus einen oder mehrere Haarbehandlungsparameter abzuleiten zum Steuern oder Regeln der Behandlungsvorrichtung, z.B. wie oben beschrieben mittels Steuerns oder Regelns einer Temperatur der Behandlungsvorrichtung, einer auf das Haar 102H des Nutzers 102 ausgeübten Kraft (z.B. in Form einer Massage, z.B. mittels eines Luftstroms und/oder mittels angetriebener Rollen), und/oder einer Dosierung eines Haarbehandlungsmittels.

Die thermischen/mechanischen Elemente der Behandlungsvorrichtung können in verschiedenen Ausführungsbeispielen jeweils mittels eines Aktuators 110 gesteuert oder geregelt sein oder werden.

Beispielsweise kann die Beleuchtungsvorrichtung 118 als Aktuator, wie oben erwähnt, eine Beleuchtungssteuerung aufweisen, die Heizvorrichtung 122 als Aktuator 110 eine Temperatursteuerung/-regelung aufweisen, das Ausüben der Kraft auf das Haar kann mittels eines steuer-/regelbaren Motors als Aktuator 110 erfolgen, und/oder die Aufbringvorrichtung (Düsen 120 der Aufbringvorrichtung sind in FIG. 1B dargestellt) für das Haarbehandlungsmittel kann als Aktuator 110 eine Pumpe und/oder ein Dosierventil aufweisen.

Der Aktuator 110 kann somit eingerichtet sein, einen Haarbehandlungsparameter zu beeinflussen. Der Aktuator 110 kann jeweils so gesteuert oder geregelt sein oder werden, dass der mittels des Aktuators 110 beeinflusste Haarbehandlungsparameter dem anhand des Haarzustandsparameters ermittelten Haarbehandlungsparameter entspricht.

Die elektronische Schaltkreisvorrichtung 104 kann in verschiedenen Ausführungsbeispielen eingerichtet sein, basierend auf den empfangenen Daten vom Sensor 106 mindestens eine Empfehlung zu ermitteln und dem Nutzer 220 bereitzustellen, beispielsweise, wie oben beschrieben, als Anleitungsvideo, als Produktempfehlung für ein bekanntes Haarbehandlungsmittel oder als Rezept für ein individuelles Haarbehandlungsmittel wie oben beschrieben.

In verschiedenen Ausführungsbeispielen, z.B. in einem Fall, in welchem der mindestens eine Sensor 106 genutzt wird, um eine Haardichte, einen Haarschädigungsgrad, eine Lockigkeit oder ähnliches zu ermitteln, kann die Behandlungsvorrichtung 100 eingerichtet sein, den Aktuator 110 zu steuern, z.B. eine der Haardichte, dem Haarschädigungsgrad, der Lockigkeit o.ä. entsprechende Beleuchtungsbehandlung zu verwirklichen, z.B. mittels des Einstellens von Beleuchtungsdauer und -position.

In verschiedenen Ausführungsbeispielen kann dem Nutzer 220 außerdem eine Haarbehandlungsempfehlung bereitgestellt werden, z.B. eine Empfehlung "jede zu behandelnde Stelle der Kopfhaut für mindestens fünf Sekunden beleuchten" oder ähnliches.

In verschiedenen Ausführungsbeispielen, wie in FIG. 1B dargestellt, kann der Aktuator 110, beispielsweise alternativ oder zusätzlich zur Temperatursteuerung oder -regelung, die steuer- oder regelbare Aufbringvorrichtung 120 (auch als Dosiervorrichtung bezeichnet) aufweisen, welche eingerichtet sein kann, das Haarbehandlungsmittel basierend auf den erfassten Sensordaten zu dosieren.

Wie oben ausgeführt, kann die Aufbringvorrichtung 120 mindestens eine Pumpe und/oder mindestens ein Ventil aufweisen, welches derart steuer- oder regelbar sein kann, dass ein Volumen oder eine Menge des Haarbehandlungsmittels dosiert werden kann. Das Dosieren kann in verschiedenen Ausführungsbeispielen in Abhängigkeit von einer Position der Behandlungsvorrichtung 100 am Haar 220H oder auf der Kopfhaut erfolgen, beispielsweise wie oben beschrieben.

Wie in FIG. 1C dargestellt ist, kann in verschiedenen Ausführungsbeispielen das Haarbehandlungsmittel in der Behandlungsvorrichtung 100 in einer Kartusche 130 aufgenommen sein oder werden. Die Kartusche 130 kann beispielsweise wiederbefüllbar sein. Die Kartusche 130 kann in verschiedenen Ausführungsbeispielen in die Behandlungsvorrichtung 100 einbringbar sein, z.B. mittels einer Öffnung 116.

Das Dosierventil kann in verschiedenen Ausführungsbeispielen Teil der Behandlungsvorrichtung 100 sein. Alternativ kann das Dosierventil Teil der Kartusche 130 sein.

In verschiedenen Ausführungsbeispielen kann, statt die Kartusche 130 zu verwenden, das Haarbehandlungsmittel direkt in einen Aufnahmeraum der Behandlungsvorrichtung 100 einbringbar sein.

In verschiedenen Ausführungsbeispielen kann das System 200 zum Verbessern eines Haarzustands über eine Mischvorrichtung 132 verfügen, mittels welcher das personalisierte Haarbehandlungsmittel (wie oben beschrieben) zubereitbar sein kann. In verschiedenen Ausführungsbeispielen kann das personalisierte Haarbehandlungsmittel mittels einer nicht zum System 200 zum Verbessern eines Haarzustands gehörenden Mischvorrichtung angefertigt und in der Kartusche bereitgestellt werden, beispielsweise in einem Kosmetiksalon, bei einem sonstigen Hersteller, z.B. bei einem Internethändler, o.ä.

Bei dem System 200a zum Verbessern eines Haarzustands aus FIG. 1A kann in verschiedenen Ausführungsbeispielen die Sensorvorrichtung, in diesem Fall der mindestens eine Sensor 106, in die beispielhaft als Haarreif ausgeführte Behandlungsvorrichtung 100, 100a integriert sein. Die Behandlungsvorrichtung 100a kann in verschiedenen Ausführungsbeispielen eingerichtet sein, mittels der integrierten elektronischen Schaltkreisvorrichtung 104 basierend auf dem mittels des integrierten Sensors 106 erfassten Haarzustandsparameter mindestens einen HaarBehandlungsparameter zu ermitteln und die Haarbehandlung mittels des mindestens einen Aktuators 110 zu steuern, wobei die Haarbehandlung ein Aufbringen des Haar-Behandlungsmittels und eine mechanische und/oder thermische Behandlung (z.B. Massieren der Kopfhaut und/oder Erhitzen oder Kühlen des Haars 220H oder der Kopfhaut) aufweist.

Das System 200b zum Verbessern eines Haarzustands aus FIG. 1B kann in verschiedenen Ausführungsbeispielen dem System 200a zum Verbessern eines Haarzustands aus FIG. 1A entsprechen, aber einen bürstenartig gestalteten Vorrichtungskörper 100K aufweisen, welcher dafür vorgesehen sein kann, am Kopf entlanggeführt zu werden während einer Behandlung. Ein Fixieren in einer Position kann in verschiedenen Ausführungsbeispielen auch möglich sein. Im Unterschied zum System 200a aus FIG. 1A kann in verschiedenen Ausführungsbeispielen die Sensorvorrichtung 114 eine von der Behandlungsvorrichtung 100b separate Vorrichtung sein, beispielsweise wie oben beschrieben.

Bei dem System 200c zum Verbessern eines Haarzustands aus FIG. 1C kann in verschiedenen Ausführungsbeispielen kann der Vorrichtungskörper 100K blockartig gestaltet sein, und ferner kann die Behandlungsvorrichtung 100c mit einer Dosiervorrichtung 100c wie oben beschrieben versehen sein. Ferner kann eine Mischvorrichtung 132 wie oben beschrieben Teil des Behandlungssystems 200c sein.

Das System 200d zum Verbessern eines Haarzustands aus FIG. 1D kann sich in verschiedenen Ausführungsbeispielen vom System 200a zum Verbessern eines Haarzustands aus FIG. 1A dahingehend unterscheiden, dass die Behandlungsvorrichtung 100c eine Datenaustauschvorrichtung 104a aufweist, welche eingerichtet sein kann, Daten kabellos zu übertragen und zu empfangen (dargestellt als Signale 224 und 230).

Das System 200 zum Verbessern eines Haarzustands, 200d kann in verschiedenen Ausführungsbeispielen eingerichtet sein, Daten mittels der Datenaustauschvorrichtung 104a mit einer externen Datenverarbeitungsvorrichtung 226 auszutauschen. Die externe Datenverarbeitungsvorrichtung kann beispielsweise ein externer Computer, z.B. eine Cloud sein.

Die externe Datenverarbeitungsvorrichtung 226 kann, wie oben beschrieben, eingerichtet sein, die erfassten Sensordaten von dem System 200d zum Verbessern eines Haarzustands zu empfangen, die Steuerungs- oder Regelungsparameter und/oder die mindestens eine Empfehlung zu ermitteln und diese dem System 200c zum Verbessern eines Haarzustands zurück zu übermitteln. Das heißt, das System 200d zum Verbessern eines Haarzustands kann, wie oben beschrieben, eingerichtet sein, die Steuerungs- oder Regelungsparameter und/oder die mindestens eine Empfehlung indirekt zu ermitteln.

Das System 200e zum Verbessern eines Haarzustands, 200e aus FIG. 1E kann in verschiedenen Ausführungsbeispielen im Wesentlichen dem System 200d zum Verbessern eines Haarzustands aus FIG. 1D entsprechen, aber um eine Ausgabevorrichtung 228 ergänzt sein. Beispielhaft ist ein Bildschirm, d.h. eine Anzeigevorrichtung als Ausgabevorrichtung 228 dargestellt. Alternativ oder zusätzlich kann das System 200e zum Verbessern eines Haarzustands allerdings über eine beliebige Art der Ausgabevorrichtung 228 verfügen, beispielsweise wie oben beschrieben.

Die Ausgabevorrichtung 228 kann in verschiedenen Ausführungsbeispielen eingerichtet sein, Daten (als (z.B. kabelloses) Signal 222) von der Behandlungsvorrichtung 100d zu empfangen und auszugeben, z.B. anzuzeigen, beispielsweise eine mittels der elektronischen Schaltkreisvorrichtung 104 und/oder der externen Datenverarbeitungsvorrichtung 226 ermittelte Empfehlung, z.B. eine Haarbehandlungsempfehlung und/oder ein empfohlenes Haarwuchs- und/oder Haarpflegemittel oder Ähnliches.

Das System 200f zum Verbessern eines Haarzustandsaus FIG. 1F kann in verschiedenen Ausführungsbeispielen eine Behandlungsvorrichtung 100, 100e aufweisen, welche wie oben beschrieben helmartig geformt sein kann und die Beleuchtungsvorrichtung 118, den Sensor 106 und die elektronische Schaltkreisvorrichtung 104 aufweisen kann.

Das System 200f kann über eine separate Ausgabevorrichtung 228 verfügen, welche wie oben beschrieben Daten von der Behandlungsvorrichtung 100f empfangen und ausgeben, z.B. darstellen kann.

Die Behandlungsvorrichtung 100e kann in verschiedenen Ausführungsbeispielen eingerichtet sein, Daten 224, 230 kabellos zu übertragen und zu empfangen.

Das System 200f zum Verbessern eines Haarzustands kann in verschiedenen Ausführungsbeispielen eine Anzeigevorrichtung 228 aufweisen.

Die Anzeigevorrichtung 228 kann in verschiedenen Ausführungsbeispielen eingerichtet sein, Daten (als Signal 222) von der Behandlungsvorrichtung 100e zu empfangen und auszugeben, z.B. wie oben bei FIG. 1E beschrieben.

Das System 200g zum Verbessern eines Haarzustands aus FIG. 1G kann im Wesentlichen dem System 200b aus FIG. 1B entsprechen, abgesehen davon, dass der Vorrichtungskörper 100K als Haarreif gestaltet ist und somit im Haar 220H im Wesentlichen in einer Position anordenbar ist, statt während eines Kämmvorgangs zum Behandeln der Kopfhaut oder des Haars genutzt zu werden.

Das System 200h zum Verbessern eines Haarzustands aus FIG. 1H kann in verschiedenen Ausführungsbeispielen eine Behandlungsvorrichtung 100, 100h aufweisen, welche in wesentlichen Teilen ähnlich oder identisch der Behandlungsvorrichtung 100g aus FIG. 1G sein kann.

Im Unterschied zum System 200g zum Verbessern eines Haarzustands aus FIG. 1G können die Behandlungsvorrichtung 100h und die externe Sensorvorrichtung 114 eingerichtet sein, ihre Daten nicht direkt miteinander auszutauschen, sondern mittels einer kombinierten Datenverarbeitungs- und Anzeigevorrichtung 226, 228 (z.B. eines Smartphones, eines Tablet, eines Laptops o.ä.). Anders ausgedrückt kann die kombinierte Datenverarbeitungs- und Anzeigevorrichtung 226, 228 als Herzstück des Systems 200g zum Verbessern eines Haarzustands fungieren. Auf der kombinierten Datenverarbeitungs- und Anzeigevorrichtung 226, 228 kann beispielsweise eine App oder ein Programm installiert sein, welches eingerichtet sein kann, das Erfassen der Sensordaten und das Steuern/Regeln der Haarbehandlungsparameter zu verwalten, ggf. selbst, z.B. alternativ oder zusätzlich zur elektronischen Schaltkreisvorrichtung 104 und/oder der elektronischen Schaltkreisvorrichtung 1040, die Steuerungs- oder Regelungsparameter und/oder die Empfehlung zu ermitteln und/oder, ggf. alternativ oder zusätzlich zur Behandlungsvorrichtung 100h, die Empfehlung bereitzustellen, z.B. mittels der Ausgabevorrichtung 228, z.B. als Anzeige oder beispielsweise akustisch, z.B. mittels eines Lautsprechers.

In verschiedenen Ausführungsbeispielen kann die kombinierte Datenverarbeitungs- und Anzeigevorrichtung 226, 228 ferner eingerichtet sein, die Sensordaten einer (weiteren) externen Datenverarbeitungsvorrichtung 226 bereitzustellen, z.B. einer Cloud, und von der (weiteren) externen Datenverarbeitungsvorrichtung 226 die von dieser empfangenen Steuerungs- oder Regelungsparameter und/oder die Empfehlung der Behandlungsvorrichtung 100f zu übermitteln und/oder die Empfehlung bereitzustellen.

In verschiedenen Ausführungsbeispielen kann zum Ermitteln eines Haarzustands, z.B. eines Haarschädigungsgrads o.ä., wie oben beschrieben eine Datenbank genutzt werden, in welcher den Sensordaten oder ggf. Kombinationen von Sensordaten, die Haarzustände (z.B. Haarschädigungsgrade) zugeordnet sein können. Die Datenbank kann vorab mittels Versuchen erstellt worden sein und z.B. in der Datenverarbeitungs- und Anzeigevorrichtung 226, 228 und/oder in mindestens einer der elektronischen Schaltkreisvorrichtungen 104, 1040 gespeichert sein, und/oder kann fortwährend, z.B. bei Nutzung einer Cloud, mittels Nutzerdaten erstellt oder ergänzt werden und dem System 200g zum Verbessern eines Haarzustands bereitgestellt werden.

In verschiedenen Ausführungsbeispielen kann die Datenbank ferner eine Behandlungs- und/oder Produktempfehlung auf Basis des Haarzustands, z.B. des Haarschädigungsgrads, bereitstellen. Bei dem Ermitteln der Behandlungs- und/oder Produktempfehlung kann ferner, beispielsweise wie oben beschrieben, weitere vom Nutzer 220 bereitgestellte Information einbezogen werden.

Die erfassten (gemessenen) Sensorwerte können, wie oben beschrieben, entweder direkt in der Behandlungsvorrichtung 100 ausgewertet werden, z.B. mittels der elektronischen Schaltkreisvorrichtung 104, oder indirekt ausgewertet werden, indem sie an eine externe Datenverarbeitungsvorrichtung 226 übertragen werden, um dort ausgewertet zu werden, z.B. wie oben beschrieben. Dabei können die Sensordaten oder Teile der Sensordaten in verschiedenen Ausführungsbeispielen ausgewertet werden mittels eines Vergleichs mit (z.B. empirisch gewonnenen) Datenbankeinträgen.

In verschiedenen Ausführungsbeispielen kann das ermittelte Haarbehandlungsmittel, z.B. Haarwuchsmittel oder Haarpflegemittel, mittels der Behandlungsvorrichtung oder des Systems zum Verbessern eines Haarzustands auf das Haar aufgebracht werden, wobei Steuerungs- oder Regelungsparameter zum Dosieren des Haarbehandlungsmittels mittels der elektronischen Schaltkreisvorrichtung 104, der elektronischen Schaltkreisvorrichtung 1040 und/oder der externen Datenverarbeitungsvorrichtung 226 und/oder der weiteren externen Datenverarbeitungsvorrichtung bereitgestellt sein oder werden können.

Das System 200j zum Verbessern eines Haarzustands aus FIG. 1J kann in verschiedenen Ausführungsbeispielen eine Behandlungsvorrichtung 100, 100j aufweisen, welche in wesentlichen Teilen ähnlich oder identisch der Behandlungsvorrichtung 100h aus FIG. 1H sein kann.

Die separate Sensorvorrichtung 114 kann, wie im Zusammenhang mit FIG. 1G bereits beschrieben, ein Smartphone, ein Tablet, einen Laptop oder Ähnliches aufweisen, beispielsweise kann die Kamera und/oder ein Mikrofon des Smartphones/Tablets/Laptops 114 als Sensor 106 oder Teil des Sensors genutzt werden. Dank eines berührungsempfindlichen Bildschirms oder einer Kombination von Tastatur und Bildschirm und/oder Maus und Bildschirm kann die Sensorvorrichtung 114 des Systems 200j zum Verbessern eines Haarzustands allerdings gleichzeitig als Eingabe- und/oder Ausgabevorrichtung 228 dienen, und ein Prozessor des Smartphones/Tablets/Laptops kann als die elektronische Schaltkreisvorrichtung 1040 der Sensorvorrichtung 114 oder als die Datenverarbeitungsvorrichtung 226 dienen, z.B. mittels einer dort installierten Software (z.B. App), und Funktionen bereitstellen wie oben für die elektronische Schaltkreisvorrichtung 1040 der Sensorvorrichtung 114 oder als die Datenverarbeitungsvorrichtung 226 beschrieben.

Ferner kann der Haarreif in verschiedenen Ausführungsbeispielen mehrteilig (hier beispielhaft zweiteilig gebildet) gestaltet sein, so dass ein Teil feststehend angeordnet sein oder werden kann, und ein weiterer Teil schwenkend entlang des Kopfes bewegbar sein kann. Damit kann ermöglicht sein, vom in einer Position feststehenden Haarreif(teil) entfernte Positionen für die Haarbehandlung mittels der Vorrichtung zugänglich zu machen.

FIG. 2A zeigt eine schematische Darstellung einer Anwendung eines Systems 200a zum Verbessern eines Haarzustands gemäß verschiedenen Ausführungsbeispielen, und FIG. 2B zeigt eine schematische Darstellung einer Anwendung eines Systems 200h zum Verbessern eines Haarzustands gemäß verschiedenen Ausführungsbeispielen.

Wie in FIG. 2A dargestellt ist, kann die Behandlungsvorrichtung 100a auf für die Behandlungsvorrichtung übliche Weise zur Haarbehandlung genutzt werden. Dargestellt ist ein Haarreif.

Im Unterschied zum herkömmlichen System zum Verbessern eines Haarzustands kann System 200 zum Verbessern eines Haarzustandsa gemäß verschiedenen Ausführungsbeispielen eingerichtet sein, während der Haarbehandlung, z.B. wie oben beschrieben, mittels mindestens eines Sensors 106 mindestens einen Sensorwert zu erfassen und mindestens einen Haarbehandlungsparameter zu steuern oder zu regeln, wobei die Haarbehandlung zumindest das Beleuchten der Kopfhaut mit Licht aufweist, wie oben beschrieben.

Wie in FIG. 2B dargestellt ist, kann das System 200h zum Verbessern eines Haarzustands für eine Haarbehandlung genutzt werden. Eine externe Sensorvorrichtung 114 kann, wie oben beschrieben, genutzt werden, um mindestens einen Haarzustandsparameter, z.B. eine Haardichte, einen Haarschädigungsgrad o.ä. zu ermitteln. Dafür kann die externe Sensorvorrichtung 114 in optischen und/oder körperlichen Kontakt mit dem Haar 220H des Nutzers 220 gebracht werden.

Wie oben beschrieben kann der mindestens eine Sensorwert und/oder ein daraus ermittelter Haarzustandsparameter und/oder ein Steuerungs- oder Regelungsparameter der kombinierten Datenverarbeitungs-/Anzeigevorrichtung 226, 228 übermittelt werden (dargestellt als Signal 222, 230), und der mindestens eine Sensorwert und/oder ein daraus ermittelter Haarzustandsparameter und/oder ein Steuerungs- oder Regelungsparameter kann der Behandlungsvorrichtung 100g übermittelt werden, z.B. zum Steuern und/oder Regeln des mindestens einen Aktuators 110 und/oder zum Anzeigen der mindestens einen Empfehlung.

Dargestellt ist hier ein Übermitteln des mindestens einen Sensorwerts und/oder eines daraus ermittelten Haarzustandsparameters und/oder eines Steuerungs- oder Regelungsparameters an die Behandlungsvorrichtung 100h vor einem Verwenden der Behandlungsvorrichtung 100h. In verschiedenen Ausführungsbeispielen kann das Übermitteln auch gleichzeitig mit dem Verwenden erfolgen.

In verschiedenen Ausführungsbeispielen können die anderen oben beschriebenen Behandlungsvorrichtungen und System zum Verbessern eines Haarzustandse sinngemäß ähnlich verwendet werden wie dies hier beispielhaft für zwei System zum Verbessern eines Haarzustandse beschrieben ist.

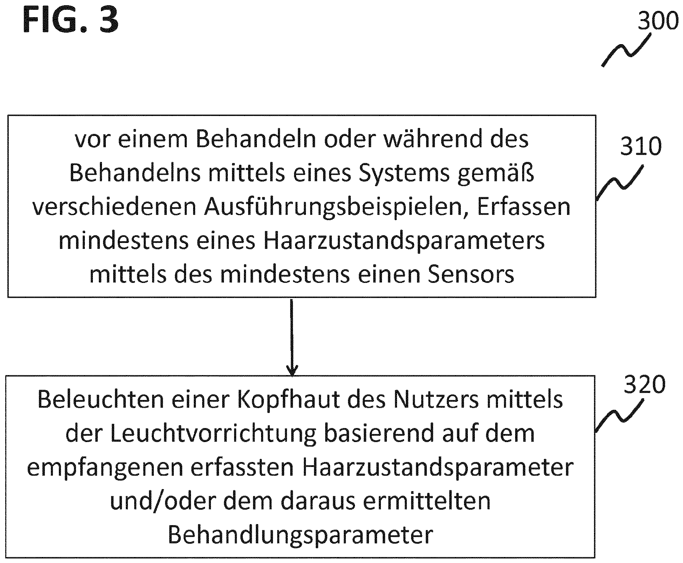

FIG. 3 zeigt ein Flussdiagramm 300 eines Verfahrens zum kosmetischen Behandeln von Haaren eines Nutzers gemäß verschiedenen Ausführungsbeispielen.

In verschiedenen Ausführungsbeispielen kann das Verfahren zum kosmetischen Behandeln von Haaren eines Nutzers aufweisen ein Erfassen mindestens eines Haarzustandsparameters vor einem Behandeln oder während des Behandelns mittels eines Systems gemäß verschiedenen Ausführungsbeispielen, mittels des mindestens einen Sensors mittels eines Systems zum Verbessern eines Haarzustands gemäß verschiedenen Ausführungsbeispielen (in 310) und ein Beleuchten einer Kopfhaut des Nutzers mittels der Leuchtvorrichtung basierend auf dem empfangenen erfassten Haarzustandsparameter und/oder dem daraus ermittelten Behandlungsparameter auf (in 320).

Für die oben beschriebenen Ermittlungen kann in verschiedenen Ausführungsbeispielen eine Programmierung, z.B. eine Software genutzt werden. Dabei kann jede Software genutzt werden, die eine oben beschriebene Funktionalität bereitstellt. In verschiedenen Ausführungsbeispielen, z.B. in einem Fall, dass zum Ausführen des Verfahrens zum kosmetischen Behandeln von Haaren eines Nutzers gemäß verschiedenen Ausführungsbeispielen ein Smartphone, ein Tablet oder ähnliches genutzt wird, kann die Software als eine App bereitgestellt sein.

In verschiedenen Ausführungsbeispielen kann die in der Behandlungsvorrichtung integrierte Schaltkreisvorrichtung und/oder eine externe Datenverarbeitungsvorrichtung, z.B. ein Smartphone, ein Tablet, ein Laptop, ein Smart Mirror, ein iPad, oder ähnliches geeignet sein, um bei einem Ausführen des Verfahrens zum Bereitstellen einer Haarzustandsinformation und/oder einer Umgebungszustandsinformation, z.B. bei Ermittlungsvorgängen, z.B. mittels Vergleichens mit einer Datenbank/Referenzwerten o.ä., verwendet zu werden. In verschiedenen Ausführungsbeispielen braucht die Programmierung/Software nicht auf dem Smartphone, dem Tablet, dem Laptop usw. bereitgestellt zu werden. Es kann beispielsweise ausreichend sein, wenn die in die Behandlungsvorrichtung integrierte Schaltkreisvorrichtung und/oder das Smartphone o.ä. durch das Internet, mittels WLAN oder auf andere gängige Weise mit einer (z.B. einer weiteren) externen Datenverarbeitungsvorrichtung, z.B. einem Computer, beispielsweise einer Cloud, verbunden ist. In einem solchen Fall können die Berechnungen beispielsweise mittels der (weiteren) externen Datenverarbeitungsvorrichtung, z.B. mittels des Computers, ausgeführt werden, und das Ergebnis kann dem Smartphone/Tablet o.ä. und/oder der internen Schaltkreisvorrichtung bereitgestellt werden, wobei die Behandlungsvorrichtung eingerichtet sein kann, die mittels der externen Datenverarbeitungsvorrichtung ermittelten Steuerungs-/Regelungsbefehle zum Steuern oder Regeln des mindestens einen Haarbehandlungsparameters einzusetzen, z.B. zum Steuern oder Regeln eines entsprechenden Aktuators.

Weitere vorteilhafte Ausgestaltungen des Verfahrens ergeben sich aus der Beschreibung der Vorrichtung und umgekehrt.

## Patentansprüche

1. System zum Verbessern eines Haarzustands, aufweisend:
eine Sensorvorrichtung mit mindestens einem Sensor zum Erfassen eines Haarzustandsparameters; und
eine Behandlungsvorrichtung, aufweisend:
eine Leuchtvorrichtung zum Beleuchten einer Kopfhaut eines Nutzers mit Licht; und
eine elektronische Schaltkreisvorrichtung, welche mit der Sensorvorrichtung gekoppelt ist zum Empfangen des erfassten Haarzustandsparameters und/oder eines mittels der Sensorvorrichtung aus dem Haarzustandsparameter ermittelten Behandlungsparameters,
wobei die elektronische Schaltkreisvorrichtung eingerichtet ist, basierend auf dem empfangenen erfassten Haarzustandsparameter und/oder dem Behandlungsparameter ein Beleuchten, mittels der Beleuchtungsvorrichtung, der Kopfhaut des Nutzers mit Licht zu steuern,
wobei die Behandlungsvorrichtung mindestens ein weiteres Behandlungselement aufweist, wobei das Behandlungselement eine Aufbringvorrichtung für ein kosmetisches Behandlungsmittel, eine Massagevorrichtung, eine Heizvorrichtung und/oder eine Kühlvorrichtung aufweist, und
wobei die elektronische Schaltkreisvorrichtung ferner eingerichtet ist, basierend auf dem empfangenen erfassten Haarzustandsparameter und/oder dem Behandlungsparameter das kosmetische Behandlungsmittel aufzubringen, eine Massage vorzunehmen, die Kopfhaut und/oder das Haar zu erwärmen und/oder die Kopfhaut und/oder das Haar zu kühlen..

2. System gemäß Anspruch 1,
wobei die Behandlungsvorrichtung einen Haarreif, einen Haarclip oder einen Helm aufweist.

3. System gemäß Anspruch 1 oder 2,
wobei der mindestens eine Sensor mindestens einen aufweist aus einer Gruppe von Sensoren, die Gruppe aufweisend:
eine Kamera zum Aufnehmen eines Bildes mittels sichtbaren Lichts, UV-Lichts und/oder Nahinfrarotlichts zum Ermitteln einer Haardichte, eines Haarschädigungsgrads, einer Lockigkeit, einer Haarfarbe und/oder einer Haardicke;
ein Mikroskop zum Ermitteln eines Haarschädigungsgrads und/oder einer Haardicke;
ein Spektrometer zum Aufnehmen eines Spektrums von sichtbarem Licht, UV-Licht und/oder Nahinfrarotlicht zum Ermitteln eines Haarschädigungsgrads und/oder eines Haarfeuchtigkeitsgehalts; und
einen akustischen Sensor zum Ermitteln eines Haarschädigungsgrads.

4. System gemäß einem der Ansprüche 1 bis 3,
wobei die Sensorvorrichtung und die Behandlungsvorrichtung separate Vorrichtungen sind.

5. System gemäß einem der Ansprüche 1 bis 3,
wobei zumindest ein Teil der Sensorvorrichtung und die Behandlungsvorrichtung eine integrierte Einheit bilden.

6. System gemäß einem der Ansprüche 1 bis 5,
wobei die elektronische Schaltkreisvorrichtung eine kabellose Datenaustauschvorrichtung aufweist.

7. System gemäß einem der Ansprüche 1 bis 6, ferner aufweisend:
eine Eingabevorrichtung und/oder eine Ausgabevorrichtung.

8. System gemäß einem der Ansprüche 1 bis 7,
wobei das Licht zum Beleuchten der Kopfhaut des Nutzers eine Wellenlänge von 640 bis 780 nm, vorzugsweise von 655 nm (+/- 5%), besitzt.

9. Verfahren zum Verbessern eines kosmetischen Haarzustands eines Nutzers, aufweisend:
vor einem Behandeln oder während des Behandelns mittels eines Systems gemäß einem der Ansprüche 1 bis 8, Erfassen mindestens eines Haarzustandsparameters mittels des mindestens einen Sensors; und
Beleuchten einer Kopfhaut des Nutzers mittels der Leuchtvorrichtung basierend auf dem empfangenen erfassten Haarzustandsparameter und/oder dem daraus ermittelten Behandlungsparameter,
wobei das Verfahren ferner aufweist:
Behandeln des Haars mittels des mindestens einen weiteren Behandlungselements basierend auf dem empfangenen erfassten Haarzustandsparameter und/oder dem daraus ermittelten Behandlungsparameter,
wobei das Behandeln mittels des mindestens einen weiteren Behandlungselements ein Aufbringen eines kosmetischen Behandlungsmittels auf das Haar oder die Kopfhaut, ein Massieren der Kopfhaut, ein Erwärmen der Kopfhaut und/oder des Haars und/oder ein Kühlen der Kopfhaut und/oder des Haars aufweist.

10. Verfahren gemäß Anspruch 9,
wobei das Ermitteln des mindestens einen Behandlungsparameters aus dem mindestens einen Haarzustandsparameter in der elektronischen Schaltkreisvorrichtung erfolgt.

11. Verfahren gemäß einem der Ansprüche 9 oder 10,
wobei das Erfassen des Haarzustands und das Beleuchten der Kopfhaut wiederholt ausgeführt werden, wobei das Erfassen des Haarzustands bei jeder wiederholten Ausführung an einer im Wesentlichen gleichen Stelle des Haars und/oder der Kopfhaut ausgeführt wird.

12. Verfahren gemäß einem der Ansprüche 9 bis 11,
wobei das kosmetische Behandlungsmittel basierend auf einer Füllstandsmenge automatisch nachbestellt wird.

## Claims

1. A system for improving a hair condition, comprising:
a sensor device having at least one sensor for detecting a hair condition parameter; and
a treatment device comprising:
a lighting device for illuminating a scalp of a user with light; and an electronic circuit device coupled to the sensor device for receiving the detected hair condition parameter and/or a treatment parameter determined from the hair condition parameter by means of the sensor device,
wherein the electronic circuit device is designed to control the illumination of the scalp of the user with light by means of the lighting device on the basis of the received detected hair condition parameter and/or the treatment parameter,
wherein the treatment device comprises at least one further treatment element, wherein the treatment element comprises an application device for a cosmetic treatment agent, a massage device, a heating device and/or a cooling device, and
wherein the electronic circuit device is also designed to apply the cosmetic treatment agent, to perform a massage, to heat the scalp and/or the hair and/or to cool the scalp and/or the hair on the basis of the received detected hair condition parameter and/or the treatment parameter.

2. The system according to claim 1,
wherein the treatment device comprises a headband, a hair clip or a helmet.

3. The system according to claim 1 or 2,
wherein the at least one sensor comprises at least one from a group of sensors, the group comprising:
a camera for recording an image by means of visible light, UV light and/or near infrared in order to determine a hair density, a degree of hair damage, curliness, a hair color and/or a hair thickness;
a microscope for determining a degree of hair damage and/or a hair thickness;
a spectrometer for capturing a spectrum of visible light, UV light and/or near infrared light in order to determine a degree of hair damage and/or a hair moisture content; and
an acoustic sensor for determining a degree of hair damage.

4. The system according to one of claims 1 to 3,
wherein the sensor device and the treatment device are separate devices.

5. The system according to one of claims 1 to 3,
wherein at least part of the sensor device and the treatment device form an integrated unit.

6. The system according to one of claims 1 to 5,
wherein the electronic circuit device comprises a wireless data exchange device.

7. The system according to one of claims 1 to 6, further comprising:
an input device and/or an output device.

8. The system according to one of claims 1 to 7,
wherein the light for illuminating the scalp of the user has a wavelength of from 640 to 780 nm, preferably 655 nm (+/- 5%).

9. A method for improving a cosmetic hair condition of a user, comprising: detecting at least one hair condition parameter by means of the at least one sensor before a treatment or during the treatment by means of a system according to one of claims 1 to 8; and
illuminating a scalp of the user by means of the lighting device on the basis of the received detected hair condition parameter and/or the treatment parameter determined therefrom,
wherein the method also comprises:
treating the hair by means of the at least one further treatment element on the basis of the received detected hair condition parameter and/or the treatment parameter determined therefrom,
wherein the treatment by means of the at least one further treatment element comprises applying a cosmetic treatment agent to the hair or the scalp, massaging the scalp, heating the scalp and/or the hair and/or cooling the scalp and/or the hair.

10. The method according to claim 9,
wherein the at least one treatment parameter is determined from the at least one hair condition parameter in the electronic circuit device.

11. The method according to one of claims 9 or 10,
wherein the detection of the hair condition and the illumination of the scalp are carried out repeatedly, wherein the detection of the hair condition in each repeated iteration is carried out at a substantially identical location of the hair and/or the scalp.

12. The method according to one of claims 9 to 11,
wherein the cosmetic treatment agent is automatically reordered based on a fill level.

## Revendications

1. Système permettant d'améliorer un état des cheveux, présentant :
un dispositif capteur comportant au moins un capteur permettant de détecter un paramètre d'état des cheveux ; et
un dispositif de traitement, présentant :
un dispositif d'éclairage permettant d'éclairer le cuir chevelu d'un utilisateur avec de la lumière ; et un dispositif de circuit électronique, lequel est couplé au dispositif capteur pour la réception du paramètre d'état des cheveux détecté et/ou d'un paramètre de traitement déterminé à l'aide du dispositif capteur à partir du paramètre d'état des cheveux,
dans lequel le dispositif de circuit électronique est configuré pour commander, sur la base du paramètre d'état des cheveux détecté reçu et/ou du paramètre de traitement, un éclairage, à l'aide du dispositif d'éclairage, du cuir chevelu de l'utilisateur avec de la lumière,
dans lequel le dispositif de traitement présente au moins un élément de traitement supplémentaire, dans lequel l'élément de traitement présente un dispositif d'application pour un agent de traitement cosmétique, un dispositif de massage, un dispositif de chauffage et/ou un dispositif de refroidissement, et
dans lequel le dispositif de circuit électronique est en outre configuré pour, sur la base du paramètre d'état des cheveux détecté reçu et/ou du paramètre de traitement, appliquer l'agent de traitement cosmétique, procéder à un massage, chauffer le cuir chevelu et/ou les cheveux et/ou refroidir le cuir chevelu et/ou les cheveux..

2. Système selon la revendication 1,
dans lequel le dispositif de traitement présente un serre-tête, une pince à cheveux ou un casque.

3. Système selon la revendication 1 ou 2,
dans lequel l'au moins un capteur présente au moins un élément parmi un groupe de capteurs, le groupe présentant :
une caméra permettant d'enregistrer une image à l'aide d'une lumière visible, d'une lumière UV et/ou d'une lumière proche infrarouge afin de déterminer une densité de cheveux, un degré d'endommagement des cheveux, une frisure, une couleur de cheveux et/ou une épaisseur de cheveux ;
un microscope permettant de déterminer un degré d'endommagement des cheveux et/ou une épaisseur de cheveux ;
un spectromètre permettant d'enregistrer un spectre de lumière visible, de lumière UV et/ou de lumière proche infrarouge afin de déterminer un degré d'endommagement des cheveux et/ou un taux d'humidité des cheveux ; et
un capteur acoustique permettant de déterminer un degré d'endommagement des cheveux.

4. Système selon l'une des revendications 1 à 3,
dans lequel le dispositif capteur et le dispositif de traitement sont des dispositifs séparés.

5. Système selon l'une des revendications 1 à 3,
dans lequel au moins une partie du dispositif capteur et le dispositif de traitement forment une unité intégrée.

6. Système selon l'une des revendications 1 à 5,
dans lequel le dispositif de circuit électronique présente un dispositif d'échange de données sans fil.

7. Système selon l'une des revendications 1 à 6, présentant en outre :
un dispositif d'entrée et/ou un dispositif de sortie.

8. Système selon l'une des revendications 1 à 7,
dans lequel la lumière permettant d'éclairer le cuir chevelu de l'utilisateur possède une longueur d'onde de 640 à 780 nm, de préférence de 655 nm (+/- 5 %).

9. Procédé permettant d'améliorer un état cosmétique des cheveux d'un utilisateur, présentant :
avant un traitement ou pendant le traitement à l'aide d'un système selon l'une des revendications 1 à 8, la détection d'au moins un paramètre d'état des cheveux à l'aide de l'au moins un capteur ; et
l'éclairage du cuir chevelu de l'utilisateur à l'aide du dispositif d'éclairage sur la base du paramètre d'état des cheveux détecté reçu et/ou du paramètre de traitement déterminé à partir de celui-ci,
dans lequel le procédé présente en outre :
le traitement des cheveux à l'aide de l'au moins un élément de traitement supplémentaire sur la base du paramètre d'état des cheveux détecté reçu et/ou du paramètre de traitement déterminé à partir de celui-ci,
dans lequel le traitement à l'aide de l'au moins un élément de traitement supplémentaire présente une application d'un agent de traitement cosmétique sur les cheveux ou le cuir chevelu, un massage du cuir chevelu, un chauffage du cuir chevelu et/ou des cheveux et/ou un refroidissement du cuir chevelu et/ou des cheveux.

10. Procédé selon la revendication 9,
dans lequel la détermination de l'au moins un paramètre de traitement est effectuée dans le dispositif de circuit électronique à partir de l'au moins un paramètre d'état des cheveux.

11. Procédé selon l'une des revendications 9 ou 10,
dans lequel la détection de l'état des cheveux et l'éclairage du cuir chevelu sont réalisés de manière répétée, dans lequel la détection de l'état des cheveux est réalisée sensiblement au même endroit des cheveux et/ou du cuir chevelu lors de chaque réalisation répétée.

12. Procédé selon l'une des revendications 9 à 11,
dans lequel l'agent de traitement cosmétique est automatiquement réapprovisionné sur la base d'un volume de remplissage.
